# EUROPEAN PATENT SPECIFICATION

(11) **EP 3 044 335 B1**
(45) Date of publication and mention of the grant of the patent: **09.09.2020**
(21) Application number: 14843312.1
(22) Date of filing: 10.09.2014
(51) Int. Cl.: C12Q 1/6886

(54) **PREDICTING BREAST CANCER RECURRENCE**
VORHERSAGE EINES BRUSTKREBSREZIDIVS
PRÉDICTION DE RÉAPPARITION DE CANCER DU SEIN

(30) Priority: 11.09.2013 US 201361876757 P
(43) Date of publication of application: 20.07.2016
(73) Proprietor: Bio Theranostics, Inc., San Diego, CA 92121 (US)
(72) Inventor: SCHNABEL, Catherine A., San Diego, CA 92121 (US); SGROI, Dennis C., San Diego, CA 92121 (US); ZHANG, Yi, San Diego, CA 92121 (US); SCHROEDER, Brock, San Diego, CA 92121 (US); ERLANDER, Mark G., San Diego, CA 92121 (US)
(74) Representative: J A Kemp LLP
(86) International application number: PCT/US2014/055041
(87) International publication number: WO 2015/038682

(56) References cited:
- WO-A1-2009/108215
- WO-A1-2013/070521
- WO-A2-2012/079059
- WO-A2-2012/079059
- US-A1- 2013 065 786
- LAUREL A HABEL ET AL: "HOXB13:IL17BR and molecular grade index and risk of breast cancer death among patients with lymph node-negative invasive disease", BREAST CANCER RESEARCH, CURRENT MEDICINE GROUP LTD, GB, vol. 15, no. 2, 14 March 2013 (2013-03-14) , page R24, XP021146267, ISSN: 1465-5411, DOI: 10.1186/BCR3402
- MA X J ET AL: "The HOXB13:IL17BR expression index is a prognostic factor in early-stage breast cancer", JOURNAL OF CLINICAL ONCOLOGY, AMERICAN SOCIETY OF CLINICAL ONCOLOGY, US, vol. 24, no. 28, 1 October 2006 (2006-10-01), pages 4611-4619, XP008101969, ISSN: 0732-183X, DOI: 10.1200/JCO.2006.06.6944
- HABEL ET AL.: 'HOXB13: IL 17 BR and molecular grade index and risk of breast cancer death among patients with lymph node-negative invasive disease' BREAST CANCER RESEARCH vol. 15, no. R24, 14 March 2013, pages 1 - 11, XP021146267
- MA ET AL.: 'A Five- Gene Molecular Grade Index and HOXB13: IL 17 BR Are Complementary Prognostic Factors in Early Stage Breast Cancer' CLIN CANCER RES vol. 14, no. 9, 01 May 2008, pages 2601 - 2608, XP055092999

## Description

### BACKGROUND OF THE INVENTION

### (1) Field of the Invention

The disclosure relates to the identification and use of gene expression profiles, or patterns, with clinical relevance to breast cancer recurrence. In particular, the disclosure provides assays for determining the likelihood of cancer recurrence after initial treatment with an anti-breast cancer therapy, such as adjuvant tamoxifen or an aromatase inhibitor.

### (2) Description of the related art

Estrogen-receptor-positive breast cancer is a disease with a protracted risk of recurrence. After 5 years of adjuvant tamoxifen, patients have a sustained risk of disease recurrence and death for at least 15 years after diagnosis. Long-term follow-up from pivotal upfront trials of adjuvant aromatase inhibitors, including the Arimidex, Tamoxifen, Alone or in Combination (ATAC) trial and Breast International Group (BIG) 1-98 study (Cuzick et al., 2010), show a continuing rate of recurrence of about 2% per year after initial therapy, with greater than half of all recurrences occurring after 5 years of adjuvant endocrine therapy. These findings emphasize the need for extended adjuvant therapy and a biomarker that can guide the treatment decision-making process.

Multigene expression signatures studied in the past decade for assessment of recurrence risk in estrogen-receptor-positive breast cancer rely mainly on the quantitative measurement of proliferation related gene expression. These multigene signatures, including the 21-gene recurrence score (Oncotype DX; Genomic Health, Redwood City, CA, USA), are strong predictors of distant recurrence, but their prognostic ability diminishes when assessing risk beyond 5 years from diagnosis (Sgroi et al., 2012). By contrast, predictors of late recurrence are not well-characterized, and different mechanisms might be associated with early and late recurrences. Biomarkers are needed to identify patients who are adequately treated with only 5 years of endocrine therapy, and conversely, those at increased risk of late recurrence who might warrant extended adjuvant endocrine or other therapy.

Previous work developed and validated the breast-cancer index (BCI) assay that consists of two independently developed gene expression biomarker sets: molecular grade index (MGI) and *HOXB13*/*IL17BR.* MGI, a five-gene predictor that recapitulates tumor grade and proliferation, is highly prognostic in patients with estrogen-receptor-positive breast cancer. *HOXB13*/*IL17BR,* which was developed independently of tumor grade or proliferation, is prognostic for early and late distant recurrences, and is predictive of extended adjuvant aromatase inhibitor benefit in patients with early-stage estrogen-receptor-positive breast cancer. Both the BCI and the 21-gene recurrence score assays measure gene expression by quantitative real-time PCR, although they differ in the genes that they detect. IHC4 is another prognostic model that measures protein expression of four of the most informative immunohistochemical biomarkers: estrogen receptors, progesterone receptors, HER2, and Ki-67 (Cusick et al., 2011), none of which are encoded by genes in the BCI assay. BCI has not been assessed in patients with estrogen-receptor-negative or triple-negative breast cancer. See US Patents 7,930,105 and 7,504,214, US Patent Publications 2011/0136680 and 2013/0281502, and PCT Patent Publication WO/2012/079059.

WO 2012/079059 discloses post-treatments for breast cancer prognosis. Laurel A Habel et al. (Breast Cancer Res. 2013 Mar 14;15(2):R24) discloses HOXB13:IL17BR and molecular grade index and risk of breast cancer death among patients with lymph node-negative invasive disease. WO 2009/108215 discloses tumor grading and cancer prognosis. Ma XJ et al. (J Clin Oncol. 2006 Oct 1;24(28):4611-9) discloses that the HOXB13:IL17BR expression index is a prognostic factor in early-stage breast cancer.

It is thus clear that there is a need for biomarkers to improve the risk-benefit of extended adjuvant endocrine therapy for late recurrence in patients with estrogen-receptor-positive breast cancer. The present invention addresses that need.

### BRIEF SUMMARY OF THE INVENTION

The present invention relates to a method of evaluating breast cancer of a subject, the method comprising measuring mRNA expression levels of HoxB13, IL17BR, Bub1B, CENPA, NEK2, RACGAP1, and RRM2 in a sample of ER+ breast cancer cells from the subject; calculating a ratio of expression levels of HoxB13/IL17BR (H:I); summing the expression levels of Bub1B, CENPA, NEK2, RACGAP1, and RRM2 using coefficients to obtain an MGI index; linearly combining H:I and MGI as continuous variables to form a Breast Cancer Index (BCI) value; and
classifying the sample, based on the BCI value, as indicating:
(i) a low risk or a high risk of cancer recurrence in the subject, with no intermediate risk category, wherein a higher BCI value is correlated with higher risk of cancer recurrence and a lower BCI value is correlated with lower risk of cancer recurrence; or
(ii) requirement or lack of requirement for treatment, wherein a lower BCI value is correlated with not requiring treatment with an aromatase inhibitor, targeted therapy or endocrine therapy after an initial treatment with an aromatase inhibitor, targeted therapy or endocrine therapy for five years or less.

The disclosure is based in part on the discovery that (a) a two category scheme (high risk, low risk) can be effectively utilized in BCI analysis to avoid the uncertainty of the prior art intermediate risk classification; (b) a linear BCI model (BCI-L) has superior prognostic ability for risk of recurrence than a cubic model (BCI-C); and (c) the above discoveries allow for a simpler and more accurate application of BCI to provide prognostic information, such as cancer recurrence, and predictive information, such as responsiveness to certain therapies, that can be used for selection of therapeutic options.

Provided herein is a method of determining risk of cancer recurrence in a subject afflicted with breast cancer. The method comprises (a) determining mRNA expression levels of a plurality of genes in a sample of ER+ breast cancer cells from the subject; and (b) classifying whether the subject has a low or high risk of cancer recurrence based on the analysis of the mRNA expression levels of the plurality of genes at diagnosis of breast cancer disease. In this method, the analysis of the plurality of genes provides a risk of cancer recurrence after receiving approximately five years of adjuvant therapy that is less than about 5% in the low risk group when compared to retrospective ER+ breast cancer patient datasets with greater than five years of outcome, or representative samples thereof. In many embodiments, the subject has received approximately five years of adjuvant therapy and is disease-free after that therapy.

Thus, in some embodiments, the present disclosure is directed to a method of determining risk of cancer recurrence in a subject afflicted with breast cancer. The method comprises
determining mRNA expression levels of *HoxB13, IL17BR, Bub1B* , *CENPA, NEK2, RACGAP1,* and *RRM2* in a sample of ER+ breast cancer cells from the subject;
summing the expression levels to form a Breast Cancer Index (BCI) value where a higher BCI value is correlated with higher risk of cancer recurrence and a lower BCI value is correlated with lower risk of cancer recurrence; and
classifying the sample, based on BCI value, as indicating a low risk or a high risk of cancer recurrence in the subject, with no intermediate risk category.

In other embodiments, the disclosure is directed to a method of determining responsiveness to treatment of a subject afflicted with breast cancer. The method comprises
determining mRNA expression levels of *HoxB13, IL17BR, Bub1B* , *CENPA, NEK2, RACGAP1,* and *RRM2* in a sample of ER+ breast cancer cells from the subject;
summing the expression levels to form a Breast Cancer Index (BCI) value, where a lower BCI value is correlated with responsiveness to additional treatment with an aromatase inhibitor, targeted therapy or endocrine therapy after an initial treatment with an aromatase inhibitor, targeted therapy or endocrine therapy for five years or less; and
classifying the sample, based on the BCI, as indicating said responsiveness or lack thereof.

Additionally, the disclosure is directed to a method of treating a subject afflicted with breast cancer. The method comprises
determining mRNA expression levels of *HoxB13, IL17BR, Bub1B* , *CENPA, NEK2, RACGAP1,* and *RRM2* in a sample of ER+ breast cancer cells from the subject;
summing the expression levels to form a Breast Cancer Index (BCI) value, where a lower BCI value is correlated with (a) responsiveness to additional treatment with an aromatase inhibitor, targeted therapy or endocrine therapy after an initial treatment with an aromatase inhibitor, targeted therapy or endocrine therapy for five years or less and (b) a lower risk of distant recurrence; and
treating the subject consistent with the BCI value determination for the subject.

### BRIEF DESCRIPTION OF THE DRAWINGS

FIG. 1 is an ATAC CONSORT diagram for the consort described herein.
FIG. 2 is graphs showing the performance of pre-specified risk groups based on BCI-C and BCI-L for overall 10-year distant recurrences in all ER+NO patients. Panel A - BCI-C; Panel B - BCI-L.
FIG. 3 is a graph showing the risk of overall 10-year distant recurrence as a function of continuous BCI-linear index in ER+ N0 patients.
FIG. 4A and 4B are graphs showing the performance of pre-specified risk groups based on BCI-C and BCI-L models for overall 10-year distant recurrence in ER+ NO HER2-negative patients. Panel A) BCI-C; 4B) BCI- L.
FIG. 5A and 5B are graphs showing the performance of BCI pre-specified risk groups for early and late distant recurrences in ER+ NO patients. 5A) early 0-5 year distant recurrence; 5B) late 5-10 year distant recurrence. Population PI refers to the pre-specified low and intermediate risk groups while P2 refers to the high risk group for early recurrence. P3 refers to the pre-specified low risk group, while P4 refers to the intermediate and high risk groups for late recurrence.
FIG. 6A and 6B are graphs showing the risk of early and late distant recurrence as a function of continuous BCI index in ER+ NO patients. 6A) risk of early 0-5 year distant recurrence; 6B) risk of late 5-10 year distant recurrence. Vertical lines delineate the borders between the low, intermediate (Inter) and high pre-specified BCI risk groups.
FIG. 7 is graphs showing the performance of BCI pre-specified risk groups for early and late distant recurrences in ER+NO HER2-negative patients. Panel A - early (0-5 years) distant recurrence; B) risk of late (5-10 years) distant recurrence.
FIG. 8 is graphs showing the risk of early (0-5 years) and late (5-10 years) distant recurrence as a function of BCI index in ER+ NO HER2-negative patients. Panel A - risk of early distant recurrence; Panel B - risk of late distant recurrence.

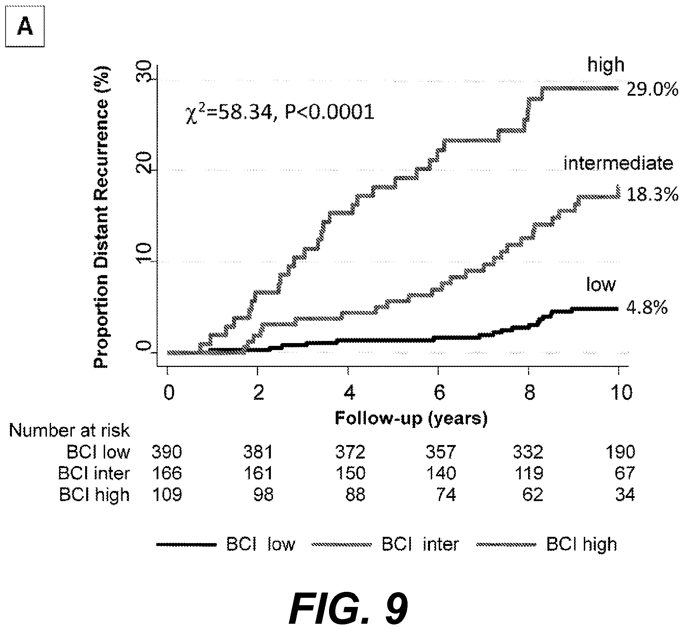

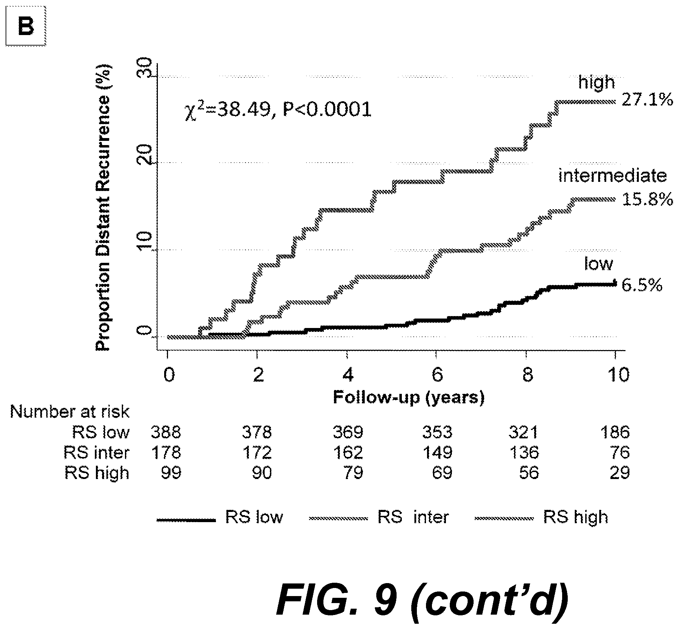

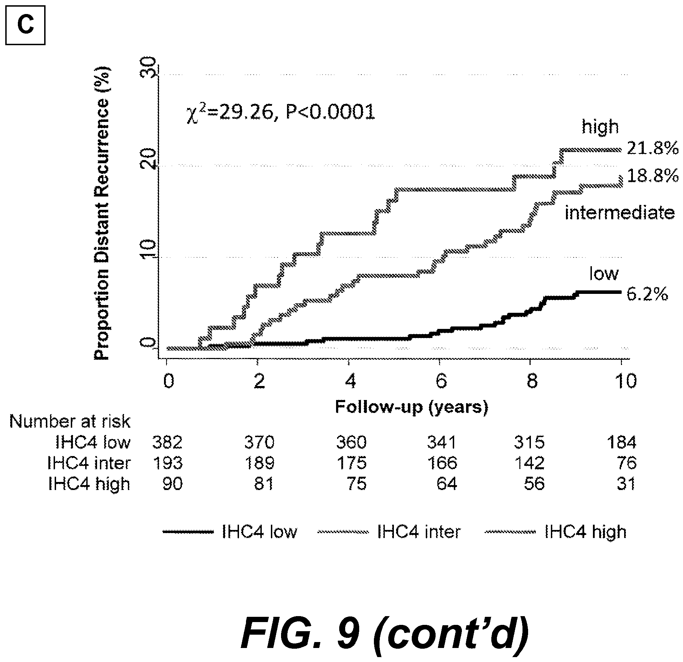

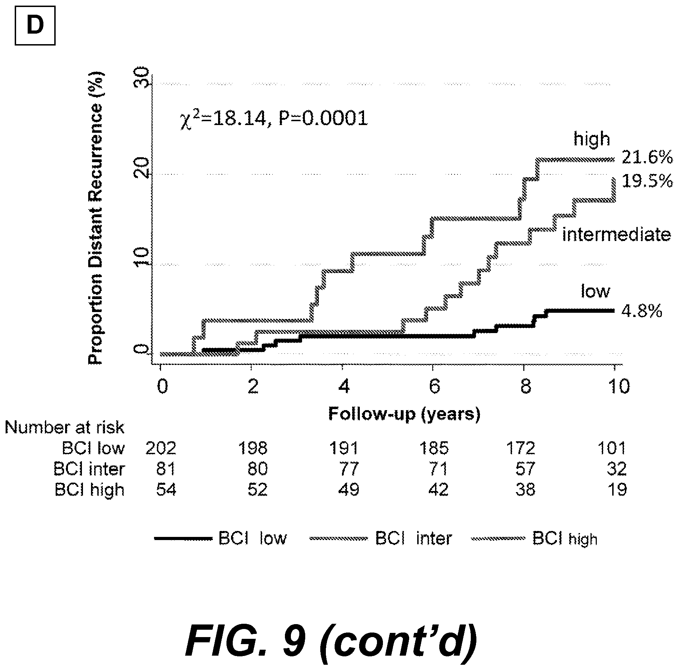

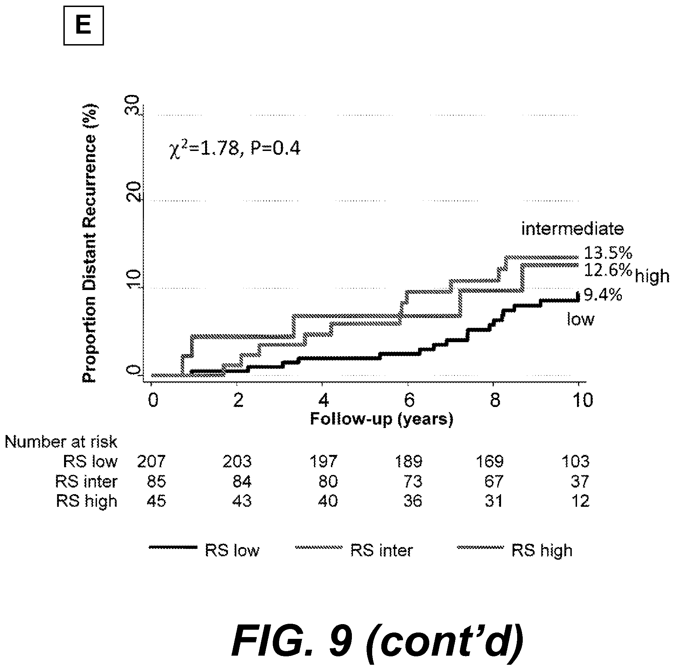

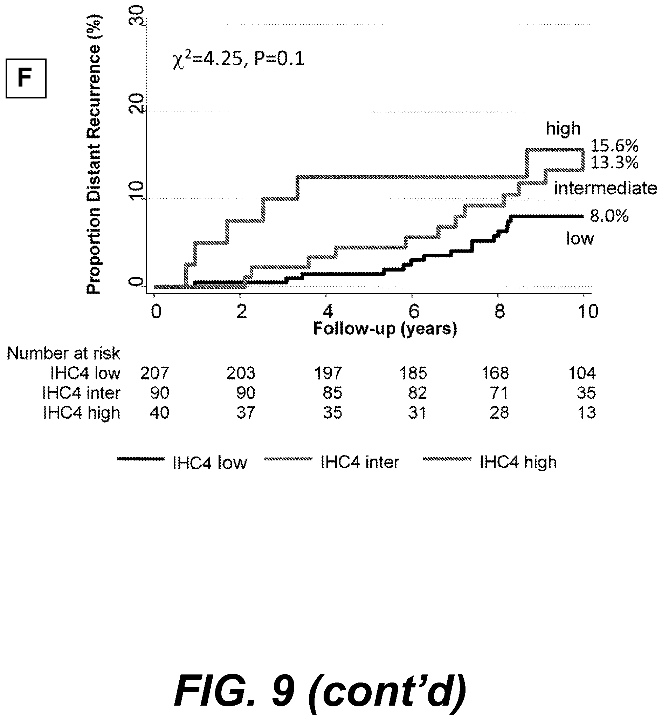

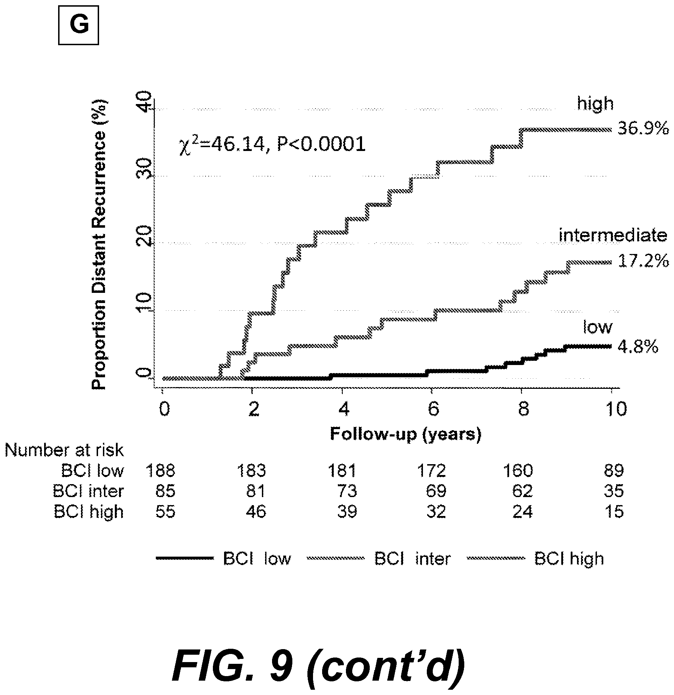

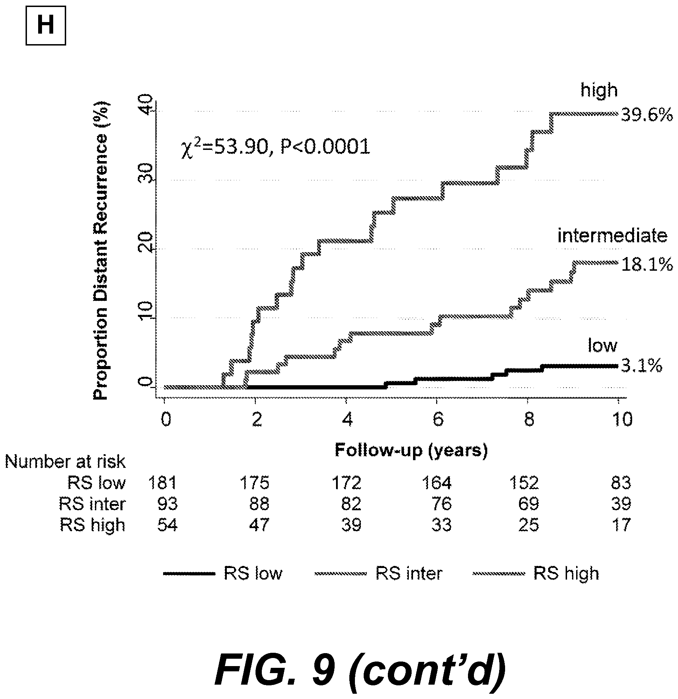

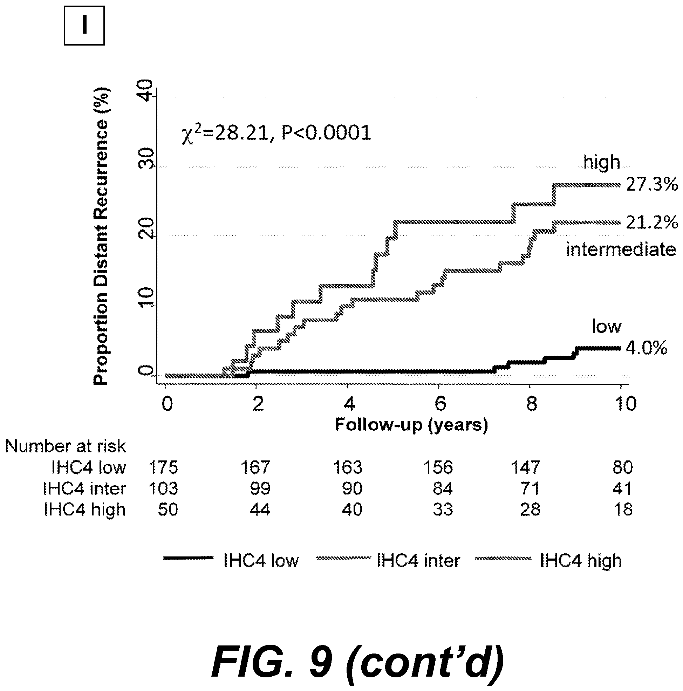

FIG. 9 is graphs showing the performance of the pre-specified risk groups of BCI and RS and the post-hoc determined categorical risk groups of IHC4 for overall 10-year distant recurrences in ER+ NO patients, both arms combined and anastrozole (ANA) and tamoxifen (TAM) arm separately. Panel A - BCI in both arms combined; Panel B - RS in both arms combined; Panel C - IHC4 in both arms combined; Panel D - BCI in anastrozole arm alone; Panel E - RS in anastrozole arm alone; Panel F - IHC4 in anastrozole arm alone; Panel G - BCI in tamoxifen arm alone; Panel H - RS in tamoxifen arm alone; Panel I - IHC4 in tamoxifen arm alone.
FIG. 10 is a graph showing the performance of pre-specified risk groups for overall 10-year distant recurrences in ER+ node-positive patients.

### DETAILED DESCRIPTION OF THE INVENTION

As used herein, the singular forms "a", "an" and "the" are intended to include the plural forms as well, unless the context clearly indicates otherwise. Additionally, the use of "or" is intended to include "and/or", unless the context clearly indicates otherwise.

### Definitions

A gene expression "pattern" or "profile" or "signature" refers to the relative expression of one or more genes between two or more clinical outcomes, cancer outcomes, cancer recurrence and/or survival outcomes which is correlated with being able to distinguish between said outcomes. In some cases, the outcome is that of breast cancer.

A "gene" is a polynucleotide that encodes a discrete product, whether RNA or proteinaceous in nature. It is appreciated that more than one polynucleotide may be capable of encoding a discrete product. The term includes alleles and polymorphisms of a gene that encodes the same product, or a functionally associated (including gain, loss, or modulation of function) analog thereof, based upon chromosomal location and ability to recombine during normal mitosis.

The terms "correlate" or "correlation" or equivalents thereof refer to an association between expression of one or more genes and a physiologic state of a cell to the exclusion of one or more other state as identified by use of the methods as described herein. A gene may be expressed at a higher or a lower level and still be correlated with one or more cancer state or outcome.

A "polynucleotide" is a polymeric form of nucleotides of any length, either ribonucleotides or deoxyribonucleotides. This term refers only to the primary structure of the molecule. Thus, this term includes double- and single-stranded DNA and RNA. It also includes known types of modifications including labels known in the art, methylation, "caps", substitution of one or more of the naturally occurring nucleotides with an analog, and internucleotide modifications such as uncharged linkages (e.g., phosphorothioates, phosphorodithioates, etc.), as well as unmodified forms of the polynucleotide.

The term "amplify" is used in the broad sense to mean creating an amplification product can be made enzymatically with DNA or RNA polymerases, for example using polymerase chain reaction (PCR), as is known in the art. "Amplification," as used herein, generally refers to the process of producing multiple copies of a desired sequence, particularly those of a sample. "Multiple copies" mean at least 2 copies. A "copy" does not necessarily mean perfect sequence complementarity or identity to the template sequence.

By corresponding is meant that a nucleic acid molecule shares a substantial amount of sequence identity with another nucleic acid molecule. Substantial amount means at least 95%, usually at least 98% and more usually at least 99%, and sequence identity is determined using the BLAST algorithm, as described in Altschul et al., J. Mol. Biol. 215:403-410 (1990) (using the published default setting, i.e. parameters w=4, t=17). Methods for amplifying mRNA are generally known in the art, and include reverse transcription PCR (RT-PCR) and those described in U.S. Patent 6,794,141. Another method which may be used is quantitative PCR (or Q-PCR). Alternatively, RNA may be directly labeled as the corresponding cDNA by methods known in the art.

A "microarray" is a linear or two-dimensional array of preferably discrete regions, each having a defined area, formed on the surface of a solid support such as, but not limited to, glass, plastic, or synthetic membrane. The density of the discrete regions on a microarray is determined by the total numbers of immobilized polynucleotides to be detected on the surface of a single solid phase support, preferably at least about 50/cm², more preferably at least about 100/ cm², even more preferably at least about 500/ cm², but preferably below about 1,000/ cm². Preferably, the arrays contain less than about 500, about 1000, about 1500, about 2000, about 2500, or about 3000 immobilized polynucleotides in total. As used herein, a DNA microarray is an array of oligonucleotides or polynucleotides placed on a chip or other surfaces used to hybridize to amplified or cloned polynucleotides from a sample. Since the position of each particular group of primers in the array is known, the identities of a sample polynucleotides can be determined based on their binding to a particular position in the microarray.

Because the disclosure relies upon the identification of genes that are over- or under-expressed, one embodiment of the disclosure involves determining expression by hybridization of mRNA, or an amplified or cloned version thereof, of a sample cell to a polynucleotide that is unique to a particular gene sequence. Preferred polynucleotides of this type contain at least about 20, at least about 22, at least about 24, at least about 26, at least about 28, at least about 30, or at least about 32 consecutive basepairs of a gene sequence that is not found in other gene sequences. The term "about" as used in the previous sentence refers to an increase or decrease of 1 from the stated numerical value. Even more preferred are polynucleotides of at least or about 50, at least or about 100, at least about or 150, at least or about 200, at least or about 250, at least or about 300, at least or about 350, or at least or about 400 basepairs of a gene sequence that is not found in other gene sequences. The term "about" as used in the preceding sentence refers to an increase or decrease of 10% from the stated numerical value. Such polynucleotides may also be referred to as polynucleotide probes that are capable of hybridizing to sequences of the genes, or unique portions thereof, described herein. Preferably, the sequences are those of mRNA encoded by the genes, the corresponding cDNA to such mRNAs, and/or amplified versions of such sequences. In preferred embodiments of the disclosure, the polynucleotide probes are immobilized on an array, other devices, or in individual spots that localize the probes.

In another embodiment of the disclosure, all or part of a disclosed sequence may be amplified and detected by methods such as the polymerase chain reaction (PCR) and variations thereof, such as, but not limited to, quantitative PCR (Q-PCR), reverse transcription PCR (RT-PCR), and real-time PCR, optionally real-time RT-PCR. Such methods would utilize one or two primers that are complementary to portions of a disclosed sequence, where the primers are used to prime nucleic acid synthesis. The newly synthesized nucleic acids are optionally labeled and may be detected directly or by hybridization to a polynucleotide of the disclosure. The newly synthesized nucleic acids may be contacted with polynucleotides (containing sequences) of the disclosure under conditions which allow for their hybridization.

Alternatively, and in another embodiment of the disclosure, gene expression may be determined by analysis of expressed protein in a cell sample of interest by use of one or more antibodies specific for one or more epitopes of individual gene products (proteins) in said cell sample. Such antibodies are preferably labeled to permit their easy detection after binding to the gene product.

The term "label" refers to a composition capable of producing a detectable signal indicative of the presence of the labeled molecule. Suitable labels include radioisotopes, nucleotide chromophores, enzymes, substrates, fluorescent molecules, chemiluminescent moieties, magnetic particles, bioluminescent moieties, and the like. As such, a label is any composition detectable by spectroscopic, photochemical, biochemical, immunochemical, electrical, optical or chemical means.

The term "support" refers to conventional supports such as beads, particles, dipsticks, fibers, filters, membranes and silane or silicate supports such as glass slides.

As used herein, a "cancer tissue sample" or "cancer cell sample" refers to a cell containing sample of tissue isolated from an individual afflicted with the corresponding cancer. The sample may be from material removed via a surgical procedure, such as a biopsy. Such samples are primary isolates (in contrast to cultured cells) and may be collected by any suitable means recognized in the art. In some embodiments, the "sample" may be collected by a non-invasive method, including, but not limited to, abrasion or fine needle aspiration.

A "breast tissue sample" or "breast cell sample" refers to a sample of breast tissue or fluid isolated from an individual suspected of being afflicted with, or at risk of developing, breast cancer. Such samples are primary isolates (in contrast to cultured cells) and may be collected by any non-invasive means, including, but not limited to, ductal lavage, fine needle aspiration, needle biopsy, the devices and methods described in U.S. Patent No. 6,328,709, or any other suitable means recognized in the art. Alternatively, the "sample" may be collected by an invasive method, including, but not limited to, surgical biopsy.

"Expression" and "gene expression" include transcription and/or translation of nucleic acid material. Of course the term may also be limited, if so indicated, as referring only to the transcription of nucleic acids.

As used herein, the term "comprising" and its cognates are used in their inclusive sense; that is, equivalent to the term "including" and its corresponding cognates.

Conditions that "allow" an event to occur or conditions that are "suitable" for an event to occur, such as hybridization, strand extension, and the like, or "suitable" conditions are conditions that do not prevent such events from occurring. Thus, these conditions permit, enhance, facilitate, and/or are conducive to the event. Such conditions, known in the art and described herein, depend upon, for example, the nature of the nucleotide sequence, temperature, and buffer conditions. These conditions also depend on what event is desired, such as hybridization, cleavage, strand extension or transcription.

Sequence "mutation," as used herein, refers to any sequence alteration in the sequence of a gene disclosed herein interest in comparison to a reference sequence. A sequence mutation includes single nucleotide changes, or alterations of more than one nucleotide in a sequence, due to mechanisms such as substitution, deletion or insertion. Single nucleotide polymorphism (SNP) is also a sequence mutation as used herein. Because the present disclosure is based on the relative level of gene expression, mutations in non-coding regions of genes as disclosed herein may also be assayed in the practice of the disclosure.

"Detection" includes any means of detecting, including direct and indirect detection of gene expression and changes therein. For example, "detectably less" products may be observed directly or indirectly, and the term indicates any reduction (including the absence of detectable signal). Similarly, "detectably more" product means any increase, whether observed directly or indirectly.

Differences in expression of the disclosed sequences between two conditions being evaluated (e.g., high or low risk of recurrence) are defined in the following terms based upon percent or fold changes in expression between the two conditions. Differences between the two conditions may be of 10, 20, 30, 40, 50, 60, 70, 80, 90, 100, 120, 140, 160, 180, or 200% .

Alternatively, fold increases or decreases from one condition to the other condition may be of 1, 1.5, 2, 2.5, 3, 3.5, 4, 4.5, 5, 5.5, 6, 6.5, 7, 7.5, 8, 8.5, 9, 9.5, or 10.

Unless defined otherwise all technical and scientific terms used herein have the same meaning as commonly understood to one of ordinary skill in the art to which this disclosure belongs.

The present invention is based in part on several discoveries further discussed below. The first discovery is that a two category scheme (high risk, low risk) can be effectively utilized in BCI analysis to avoid the uncertainty of the prior art intermediate risk classification. See, e.g., Example 2 below. The second discovery is that a linear BCI model (BCI-L) has superior prognostic ability for risk of recurrence than a cubic model (BCI-C). These discoveries allow for a simpler and more accurate application of BCI to provide prognostic information, such as cancer recurrence, and predictive information, such as responsiveness to certain therapies, that can be used for selection of therapeutic options. As such, BCI could identify a low risk group having a risk of recurrence of less than 5% that encompasses more than 60% of breast cancer patients (Example 2). This gives more than 60% of patients undergoing initial therapy to forgo extended therapy with little risk of recurrence.

These advantages are provided herewith as methods of determining prognosis and/or cancer recurrence by assaying for the expression patterns disclosed herein. So where subjective interpretation may have been previously used to determine the prognosis and/or treatment of cancer patients, this disclosure provides objective gene expression patterns, which may be used alone or in combination with subjective criteria to provide a more accurate assessment of patient outcomes, including survival and the recurrence of cancer.

Thus, in some embodiments, a method of determining risk of cancer recurrence in a subject afflicted with breast cancer is provided. The method comprises (a) determining mRNA expression levels of a plurality of genes in a sample of ER+ breast cancer cells from the subject; and (b) classifying whether the subject has a low or high risk of cancer recurrence based on the analysis of the mRNA expression levels of the plurality of genes at diagnosis of breast cancer disease. In this method, the analysis of the plurality of genes provides a risk of cancer recurrence after receiving approximately five years of adjuvant therapy that is less than about 5% in the low risk group when compared to retrospective ER+ breast cancer patient datasets with greater than five years of outcome, or representative samples thereof. In many embodiments, the subject has received approximately five years of adjuvant therapy and is disease-free after that therapy.

These methods can be practiced using only a low and high risk classification. In alternative embodiments, an intermediate risk category is also classified.

In some embodiments, the low risk group comprises more than 50% of the dataset. In other embodiments, the low risk group comprises more than 55% of the dataset. In additional embodiments, the low risk group comprises more than 60% of the dataset.

These methods can assess the risk of recurrence for any time period, e.g., 5 years or less, greater than 5 years, at 10 years, greater than 10 years, etc. Additionally, these methods can assess the risk of distant recurrence or local recurrence, or both.

Any plurality of genes can be used for these methods, provided that, when their expression levels are analyzed, they are able to identify a low risk group having a less than 5% risk of recurrence. In some embodiments, one of the plurality of genes is *HoxB13.* In other embodiments, one of the plurality of genes is *IL17BR.* In further embodiments, the ratio of expression levels of *HoxB13*/*IL17BR* (H:I) is determined.

In additional embodiments, the plurality of genes comprise 1, 2, 3, 4 or all 5 of the MGI genes, *Bub1B, CENPA, NEK2, RACGAP1,* and *RRM2.* In some embodiments, the expression levels of *Bub1B, CENPA, NEK2, RACGAP1,* and *RRM2* are determined. In some of those embodiments, the ratio of expression levels of *HoxB13*/*IL17BR* (H:I) is also determined. In various aspects of those embodiments, the expression levels of *Bub1B, CENPA, NEK2, RACGAP1,* and *RRM2* are summed and a coefficient applied to obtain an MGI index, and H:I and MGI are combined as continuous variables into a BCI value.

Where BCI is used, the BCI may be calculated in any manner known in the art. In some embodiments, BCI is calculated by assessing the individual risk of cancer recurrence as part of a continuous BCI variable, wherein the risk of recurrence increases in a linear relationship with the BCI variable.

The present disclosure is also directed to a method of determining risk of cancer recurrence in a subject afflicted with breast cancer. The method comprises
determining mRNA expression levels of *HoxB13, IL17BR, Bub1B* , *CENPA, NEK2, RACGAP1,* and *RRM2* in a sample of ER+ breast cancer cells from the subject;
summing the expression levels to form a Breast Cancer Index (BCI) value where a higher BCI value is correlated with higher risk of cancer recurrence and a lower BCI value is correlated with lower risk of cancer recurrence; and
classifying the sample, based on BCI value, as indicating a low risk or a high risk of cancer recurrence in the subject, with no intermediate risk category.

Some of the methods of the disclosure are based on the expression levels of certain genes, including the expression level of *HoxB13,* in breast cancer cells of a subject as a component of the BCI. In some embodiments, a two-gene ratio of *HoxB13* expression to *IL17BR* expression (or *HoxB13:IL17BR* ratio) is used (US Patent Application Publications 2005/0239079, 2005/0239083, and 2006/0154267). In alternative embodiments of a breast cancer index, a two-gene ratio of *HoxB13* expression to *CHDH* expression may be used.

The *HoxB13:IL17BR* (H:I) ratio was discovered based upon a study of novel biomarkers predictive of clinical outcome beyond standard prognostic factors. Patients who developed cancer recurrences were matched to those who did not with respect to tumor stage and grade. The simple H:I ratio was found to be suitable for predicting cancer recurrence in patients with estrogen receptor-positive (ER+) breast cancer receiving adjuvant tamoxifen therapy. Subsequent studies (Ma et al., 2006; Goetz et al., 2006; Jerevall et al., 2007; Jansen et al., 2007) have further shown that the ratio is both prognostic, such as by being an indicator of tumor aggressiveness, and predictive of tamoxifen benefit (*i.e.,* tamoxifen response/resistance) within both retrospective and randomized clinical trials.

The BCI includes expression of one or more additional genes in combination with *HoxB13*/*IL17BR* expression. The combination may be with any one, two, three, four or all five of the additionally disclosed genes as follows. These additional genes of the disclosure encode *Bub1B* ("budding uninhibited by benzimidazoles I beta) or p21 protein-activated kinase 6 (PAK6); *CENPA* (centromere protein A, isoform a); *NEK2* (NIMA-related kinase 2 or "never in mitosis gene a"-related kinase 2); *RACGAP1* (Rac GTPase activating protein 1); and *RRM2* (ribonucleotide reductase M2). The use of these five genes alone is referred to herein as the Molecular Grade Index (MGI). Methods of calculating MGI are discussed, e.g., in Example 2 below and US Patent Publication 2011/0136680. In some embodiments, MGI is calculated by summing the expression levels of *Bub1B, CENPA, NEK2, RACGAP1,* and *RRM2* using coefficients for each gene's expression level. The coefficients can be determined by any method known in the art. In various embodiments, the coefficients are determined from principal component analysis.

Aspects of the disclosure include compositions and methods described for the use of *HoxB13* expression, with *ILI7BR* expression, in combination with expression level(s) of one or more of the above five genes to study, to provide prognostic information, and/or provide predictions of clinical responsiveness.

The 5 MGI genes have roles in the cell cycle and reported peak expression as follows:

| Gene | Peak of Expression | Role in Cell Cycle |
|---|---|---|
| *Bub1B* | G2/M | mitotic spindle assembly checkpoint |
| *CENPA* | G2/M | centromere assembly |
| *NEK2* | G2/M | centromere duplication |
| *RACGAP1* | Not Determined | Initiation of cytokinesis |
| *RRM2* | S | DNA replication |

See PCT patent application WO/2012/079059 for details of the identity of these genes.

Thus the disclosure is based in part on the discovery that gene expression level(s) are useful for providing prognostic and predictive determinations for a subject. The use of all seven disclosed genes is referred to as the Breast Cancer Index (BCI).

As demonstrated in the Examples, BCI provides superior stratification of risk of recurrence in breast cancer patients by assigning subjects with intermediate-risk to either low-risk or high-risk, during an initial period up to five years of endocrine therapy, targeted therapy or treatment with an aromatase inhibitor. BCI is thus advantageous over other contemporary gene-expression signatures because the identification of two rather than three distinct risk groups in each time period (by grouping intermediate and low risk together for early recurrence and intermediate and high risk together for late recurrence) allows for the elimination of the intermediate-risk category that can account for as many as 40% of patients with estrogen-receptor positive breast cancer. In some cases, BCI is applied in the setting of late disease recurrence because it permits a means of identifying patients who may be spared extended adjuvant endocrine therapy and its adverse side effects.

Clinicopathological factors such as nodal status and tumor size are associated with a higher risk of late recurrence; however, the results disclosed herein represent a refinement, allowing for individualized assessment of late recurrence risk, and providing a statistically significant improvement in prognostic performance beyond clinicopathological factors. Put differently, the methods of the disclosure may be practiced without the use of, or optionally in in conjunction with, the use of clinicopathological factors such as nodal status and tumor size. Other gene-expression-based assays (EndoPredict and PAM 50) have prognostic ability for late recurrence beyond clinicopathological factors. These studies further validate the clinical use of molecular-based assays for the assessment of late disease recurrence risk.

To determine the expression levels of genes in the practice of the present disclosure, any method known in the art may be utilized. In some embodiments, expression based on detection of mRNA which hybridizes to the genes identified and disclosed herein is used. This is readily performed by any mRNA detection or amplification+detection method known or recognized as equivalent in the art such as, but not limited to, reverse transcription PCR, the methods disclosed in U.S. Patent 6,794,141, and methods to detect the presence, or absence, of RNA stabilizing or destabilizing sequences. In various embodiments, the mRNA is converted into cDNA.

The ability to discriminate is conferred by the identification of expression of the individual genes as relevant and not by the form of the assay used to determine the actual level of expression. An assay may utilize any identifying feature of an identified individual gene as disclosed herein as long as the assay reflects, quantitatively or qualitatively, expression of the gene in the "transcriptome" (the transcribed fraction of genes in a genome) or the "proteome" (the translated fraction of expressed genes in a genome). Identifying features include, but are not limited to, unique nucleic acid sequences used to encode (DNA), or express (RNA), said gene or epitopes specific to, or activities of, a protein encoded by said gene. All that is required is the identity of the gene(s) necessary to discriminate between cancer outcomes and an appropriate cell containing sample for use in an expression assay. Similarly, the nature of the cell containing sample is not limiting, as fresh tissue, freshly frozen tissue, and fixed tissue, such as formalin-fixed paraffin-embedded (FFPE) tissues, may be used in the disclosed methods.

Expression based on detection of a presence, increase, or decrease in protein levels or activity may also be used. Detection may be performed by any immunohistochemistry (IHC) based, blood based (especially for secreted proteins), antibody (including autoantibodies against the protein) based, exfoliate cell (from the cancer) based, mass spectroscopy based, and image (including used of labeled ligand) based method known in the art and recognized as appropriate for the detection of the protein. Antibody and image based methods are additionally useful for the localization of tumors after determination of cancer by use of cells obtained by a non-invasive procedure (such as ductal lavage or fine needle aspiration), where the source of the cancerous cells is not known. A labeled antibody or ligand may be used to localize the carcinoma(s) within a patient.

One embodiment using a nucleic acid based assay to determine expression is by immobilization of one or more sequences of the genes identified herein on a solid support, including, but not limited to, a solid substrate such as an array or to beads or bead-based technology as is known in the art. Alternatively, solution based expression assays known in the art may also be used.

The immobilized gene(s) may be in the form of polynucleotides that are unique or otherwise specific to the gene(s) such that the polynucleotide would be capable of hybridizing to a DNA or RNA corresponding to the gene(s). These polynucleotides may be the full length of the gene(s) or be short sequences of the genes (up to one nucleotide shorter than the full length sequence known in the art, e.g., by deletion from the 5' or 3' end of the sequence) that are optionally minimally interrupted (such as by mismatches or inserted non-complementary basepairs) such that hybridization with a DNA or RNA corresponding to the gene(s) is not affected. In some cases, the polynucleotides used are from the 3' end of the gene, such as within about 350, about 300, about 250, about 200, about 150, about 100, or about 50 nucleotides from the polyadenylation signal or polyadenylation site of a gene or expressed sequence.

The skilled person is fully capable of aligning any two or more of the known expressed sequences for each of these genes to identify an area of identity or conserved changes as a region that uniquely identifies each of these genes in comparison to other genes. Furthermore, the skilled person is fully capable of aligning any two or more of the known expressed sequences for each of these genes to identify an area unique to one or more of the of the expressed sequences as a region that uniquely identifies one known expressed sequence relative to at least one other expressed sequence. As a non-limiting example, a unique region may be in a variant of the expressed sequence for one of the known genes such that the region may be used to identify expression of the variant.

The sequences of the same genes have also been identified and characterized from other animal species. Thus the skilled person in the field is clearly aware of how to identify the disclosed genes relative to other animal genes. The skilled person may also optionally compare the known sequences of the disclosed genes from different animal sources to identify conserved regions and sequences unique to these genes relative to other genes.

Polynucleotides containing mutations relative to the sequences of the disclosed genes may also be used so long as the presence of the mutations still allows hybridization to produce a detectable signal. The immobilized gene(s) may be used to determine the state of nucleic acid samples prepared from sample cancer, or breast, cell(s) for which the outcome of the sample's subject (e.g. patient from whom the sample is obtained) is not known or for confirmation of an outcome that is already assigned to the sample's subject. Without limiting the disclosure, such a cell may be from a patient with ER+ breast cancer. The immobilized polynucleotide(s) need only be sufficient to specifically hybridize to the corresponding nucleic acid molecules derived from the sample under suitable conditions.

As will be appreciated by those skilled in the art, some of the corresponding sequences noted above include 3' polyA (or polyT on the complementary strand) stretches that do not contribute to the uniqueness of the disclosed sequences. The disclosure may thus be practiced with sequences lacking the 3' polyA (or polyT) stretches. The uniqueness of the disclosed sequences refers to the portions or entireties of the sequences which are found only in the disclosed gene's nucleic acids, including unique sequences found at the 3' untranslated portion of the genes. Preferred unique sequences for the practice of the disclosure are those which contribute to the consensus sequences for each of the three sets such that the unique sequences will be useful in detecting expression in a variety of individuals rather than being specific for a polymorphism present in some individuals. Alternatively, sequences unique to an individual or a subpopulation may be used. The preferred unique sequences are preferably of the lengths of polynucleotides of the disclosure as discussed herein.

Methods to identify increased RNA stability (resulting in an observation of increased expression) or decreased RNA stability (resulting in an observation of decreased expression) may also be used. These methods include the detection of sequences that increase or decrease the stability of mRNAs containing the genes' sequences.

These methods also include the detection of increased mRNA degradation. In some embodiments of the disclosure, polynucleotides having sequences present in the 3' untranslated and/or non-coding regions of the above disclosed sequences are used to detect expression levels of the gene sequences in cancer, or breast, cells. Such polynucleotides may optionally contain sequences found in the 3' portions of the coding regions of the above disclosed sequences.

Polynucleotides containing a combination of sequences from the coding and 3' non-coding regions preferably have the sequences arranged contiguously, with no intervening heterologous sequences.

Alternatively, the disclosure may be practiced with polynucleotides having sequences present in the 5' untranslated and/or non-coding regions of the gene sequences in cancer, or breast, cells to detect their levels of expression. Such polynucleotides may optionally contain sequences found in the 5' portions of the coding regions. Polynucleotides containing a combination of sequences from the coding and 5' non-coding regions preferably have the sequences arranged contiguously, with no intervening heterologous sequences. The disclosure may also be practiced with sequences present in the coding regions of the disclosed gene sequences.

Non-limiting polynucleotides contain sequences from 3' or 5' untranslated and/or non-coding regions of at least about 20, at least about 22, at least about 24, at least about 26, at least about 28, at least about 30, at least about 32, at least about 34, at least about 36, at least about 38, at least about 40, at least about 42, at least about 44, or at least about 46 consecutive nucleotides. The term "about" as used in the previous sentence refers to an increase or decrease of 1 from the stated numerical value. Even more preferred are polynucleotides containing sequences of at least or about 50, at least or about 100, at least about or 150, at least or about 200, at least or about 250, at least or about 300, at least or about 350, or at least or about 400 consecutive nucleotides. The term "about" as used in the preceding sentence refers to an increase or decrease of 10% from the stated numerical value.

Sequences from the 3' or 5' end of the above described coding regions as found in polynucleotides of the disclosure are of the same lengths as those described above, except that they would naturally be limited by the length of the coding region. The 3' end of a coding region may include sequences up to the 3' half of the coding region. Conversely, the 5' end of a coding region may include sequences up the 5' half of the coding region. Of course the above described sequences, or the coding regions and polynucleotides containing portions thereof, may be used in their entireties.

Polynucleotides combining the sequences from a 3' untranslated and/or non-coding region and the associated 3' end of the coding region may be at least or about 100, at least about or 150, at least or about 200, at least or about 250, at least or about 300, at least or about 350, or at least or about 400 consecutive nucleotides. Preferably, the polynucleotides used are from the 3' end of the gene, such as within about 350, about 300, about 250, about 200, about 150, about 100, or about 50 nucleotides from the polyadenylation signal or polyadenylation site of a gene or expressed sequence. Polynucleotides containing mutations relative to the sequences of the disclosed genes may also be used so long as the presence of the mutations still allows hybridization to produce a detectable signal.

In another embodiment of the disclosure, polynucleotides containing deletions of nucleotides from the 5' and/or 3' end of the above disclosed sequences may be used. The deletions are preferably of 1-5, 5-10, 10-15, 15-20, 20-25, 25-30, 30-35, 35-40, 40-45, 45-50, 50-60, 60-70, 70-80, 80-90, 90-100, 100-125, 125-150, 150-175, or 175-200 nucleotides from the 5' and/or 3' end, although the extent of the deletions would naturally be limited by the length of the sequences and the need to be able to use the polynucleotides for the detection of expression levels.

Other polynucleotides of the disclosure from the 3' end of the above disclosed sequences include those of primers and optional probes for quantitative PCR. In some embodiments, the primers and probes are those which amplify a region less than about 350, less than about 300, less than about 250, less than about 200, less than about 150, less than about 100, or less than about 50 nucleotides from the from the polyadenylation signal or polyadenylation site of a gene or expressed sequence.

In yet other embodiments of the disclosure, polynucleotides containing portions of the above disclosed sequences including the 3' end may be used. Such polynucleotides would contain at least or about 50, at least or about 100, at least about or 150, at least or about 200, at least or about 250, at least or about 300, at least or about 350, or at least or about 400 consecutive nucleotides from the 3' end of the disclosed sequences.

The disclosure also includes polynucleotides used to detect gene expression in breast cancer cells. The polynucleotides may comprise a shorter polynucleotide consisting of sequences found in the above genes in combination with heterologous sequences not naturally found in combination with the sequences. Non-limiting examples include short sequences from cloning vectors or present in restriction fragments used to prepare labeled probes or primers as described herein.

The requisite level of expression may be that which is identified by the methods described herein for the genes used. Additionally, the assaying may include preparing RNA from the sample, optionally for use in PCR (polymerase chain reaction) or other analytical methodology as described herein. The PCR methodology is optionally RT-PCR (reverse transcription-PCR) or quantitative PCR, such as real- time RT-PCR. Alternatively, the assaying may be conducted by use of an array, such as a microarray, by next-generation sequencing, or by any other method known in the art. Optionally, the sample of cancer cells is dissected from tissue removed or obtained from said subject. As described herein, a variety of sample types may be used, including a formalin fixed paraffin embedded (FFPE) sample as a non-limiting example. And as described herein, the method may include assaying or determining the H:I ratio (ratio of *HoxB13* and *IL17BR* expression levels) in the sample as disclosed herein.

By way of non-limiting example, all five genes of the MGI may be assayed and used to detect expression levels that correspond to a value that is "high risk" (which is above the cutoff) for MGI, or to detect expression levels that correspond to a value that is "low risk" (which is at or below the cutoff) for MGI, as disclosed herein. In some cases, the MGI cutoff threshold may be 0 (zero), such as where the measurements of expression levels are standardized to 0 (zero) with a standard deviation of 1. In alternative embodiments, the cutoff may be at or about 0.05, at or about 0.10, at or about 0.15, at or about 0.20, at or about 0.25, at or about -0.05, at or about -0.10, at or about -0.15, at or about -0.20, at or about -0.25, at or about -0.30, at or about -0.35, at or about -0.40, at or about -0.45, at or about -0.50, at or about -0.55, at or about -0.60, at or about -0.65, at or about -0.70, at or about -0.75, at or about-0.80, at or about -0.85, at or about -0.90, at or about -0.95, at or about -1.0, at or about -1.1, at or about -1.2, at or about -1.3, at or about - 1.4, at or about -1.5, at or about -1.6, at or about -1.7, at or about -1.8, at or about -1.9, at or about -2.0 or lower. With respect to the H:I ratio, its determination maybe made as described in Ma et al., 2004 and Ma et al., 2006. For example, a value of 0.06 may be used to determine whether a sample has a "high risk" (>0.06) or "low risk" (0.06) H:I ratio.

So using a threshold, or cutoff, of 0 (zero) as a non-limiting example for MGI with all five genes, the disclosed methods provide two possible assay outcomes for a given sample: "high risk MGI" corresponding to a value above 0 (zero) and "low risk MGI" corresponding to a value of 0. A "high risk MGI" is indicative of a "high risk" cancer, including breast cancer that is analogous to that of a Grade III tumor as defined by methodologies and standards known in the field. A "low risk MGI" is indicative of a "low risk" cancer, including breast cancer, that is analogous to that of a Grade I tumor as defined by methodologies and standards known in the field.

The threshold or cutoffs used to determine intermediate-risk of cancer recurrence may be those disclosed herein or within about 2%, 4%, 6%, 8%, 10%, 12%, 14%, 16%, 18%, 20%, 25%, 30%, 35%, 40%, 50%, 60%, 70%, 80%, 90%, 100% or more thereof.

As a non-limiting example, the cancer cell may be one from a pre-operative histological sample, or biopsy, used to diagnose cancer in the subject. For such a subject with ductal carcinoma in situ (DCIS), the current standard of care is surgery, with breast conserving surgery preferred over a radical mastectomy, to remove the DCIS. This is often followed by post-operative radiotherapy, optionally with endocrine therapy, such as treatment with tamoxifen, a selective estrogen receptor modulator (SERM), a selective estrogen receptor down-regulator (SERD), an aromatase inhibitor (AI) such as letrozole, a targeted therapy such as anti-mTOR therapy (e.g., with Afinitor®) or anti-HER2 therapy (e.g., with Herceptin®) and/or chemotherapy, using any compound known in the art.

The detection of gene expression and determination of BCI may of course be in any suitable cell containing sample as described herein. Non-limiting examples of cells for use in the disclosure include those freshly isolated from the subject, those frozen after isolation, and those that are fixed and/or embedded, such as formalin fixed, paraffin embedded (FFPE). In most embodiments, the cells are breast cells, such as breast cancer cells.

As disclosed herein, the BCI is used to determine risk of cancer recurrence in a breast cancer afflicted patient. Non-limiting examples of late recurrence include after 5 years of treatment with an aromatase inhibitor, targeted therapy or endocrine therapy, such as tamoxifen, but also includes after 4 years, after 3 years, or after 2 years or less time of treatment. Similarly, the BCI may be used to predict responsiveness to an anti-aromatase therapy, such as anastrozole or letrozole, targeted therapy or anti-estrogen therapy after the above time periods.

In some embodiments, the methods disclosed herein can be advantageously used on a breast cancer cell-containing sample from a subject, such as a DCIS sample, although the methods described herein can be used with any type of breast cancer, including any non-invasive, or invasive breast cancer, such as invasive ductal carcinoma, invasive lobular carcinoma, inflammatory breast cancer, male breast cancer, metastatic breast cancer, recurrent breast cancer, papillary carcinoma, triple-negative breast cancer, Paget's disease of the nipple, sarcoma of the breast, medullary carcinoma, tubular carcinoma, mucinous carcinoma, metaplastic carcinoma, adenocystic carcinoma, phyllodes tumor and angiosarcoma.

As discussed in Example 2 below, the risk of recurrence using BCI can be categorized as low risk and high risk, without an intermediate risk category, with no loss of accuracy. In this scheme, the intermediate classification, as described, e.g., in US Patent Publication 2013/0281502 (see, e.g., Table 2 therein), can be grouped with the low risk group when the risk of recurrence at 5 years or less (for example, 4, 3, 2 or 1 year) is classified, and can be grouped with the high risk group when the risk of recurrence at more than 5 years (for example, 6, 7, 8, 9, 10, 11, 12, 13, 14, 15 or more years) is classified.

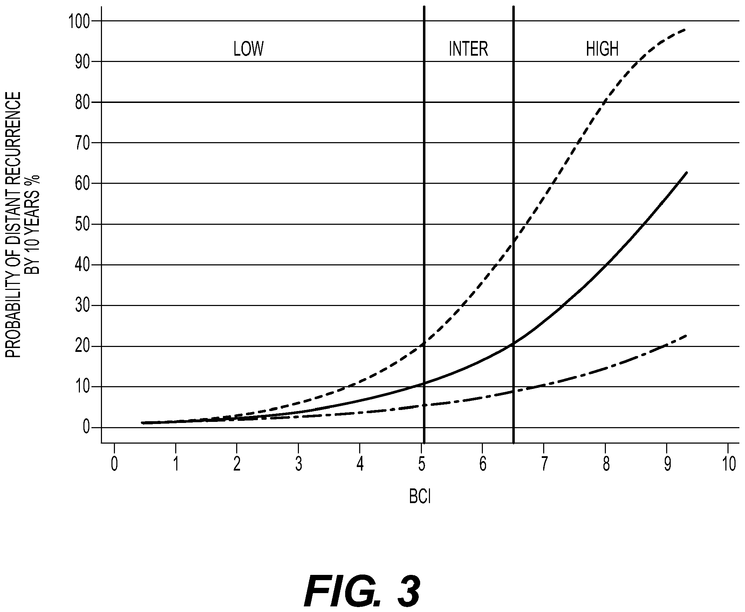

When BCI is scaled to a range of 1-10 (see, e.g., FIGS. 3, 6 and 8), the intermediate group is between BCI scores of about 5-6.5. As discussed above, when the BCI is scaled to a range of 1-10 and risk of recurrence at 5 years or less is classified, the intermediate group can be joined with the low risk group. The cutoff value between the low (+ intermediate) risk and the high risk groups is therefore where the intermediate group meets the high risk group, i.e., about 6.5, for example 5.5, 5.6, 5.7, 5.8, 5.9, 6.0, 6.1, 6.2, 6.3, 6.4, 6.6, 6.7, 6.8, 6.9, 7.0, 7.1, 7.2, 7.33, 7.4 or 7.5, or any value in between. It is understood that the cutoff value under these circumstances can be between 5.5 and 7.5, depending on how many years out the risk of recurrence is determined (fewer years allows for a higher cutoff point) and how conservative the operator wishes to be in declaring a patient as having a low or high risk of recurrence (i.e., the percentage recurrence under which the index is at a low risk of recurrence). The skilled artisan could determine a proper BCI score, without undue experimentation, for any particular number of years and level of risk desired.

Similarly, when BCI is scaled to a range of 1-10 and risk of recurrence at more than 5 years is classified, the intermediate group can be joined with the high risk group, making the cutoff between the low risk group and the high (+ intermediate) group the point at where the intermediate group meets the low risk group, i.e., about 5, for example 4.0, 4.1, 4.2, 4.3, 4.4, 4.5, 4.6, 4.7, 4.8, 4.9, 5.1, 5.2, 5.3, 5.4, 5.5, 5.6, 5.7, 5.8, 5.9, or 6.0, or any value in between.

The above specific scenarios illustrate that, for patients at low risk after either upfront (initial) treatment with tamoxifen, targeted therapy or aromatase inhibitor following breast cancer diagnosis and/or surgical intervention, BCI testing can provide the option of no further systemic therapy. For example, patients at high risk of recurrence after initial adjuvant tamoxifen (patients with high *HOXBI3*/*TI17BR*) benefit from extended hormonal therapy with a switch to the aromatase inhibitor letrozole. Those patients would exhibit a high H:I ratio or BCI score. Patients at high risk of recurrence after 5 years of initial aromatase inhibitor, targeted therapy or endocrine therapy however, may or may not benefit from extended adjuvant hormonal therapy or indeed from any systemic therapy. Those patients may also be candidates for experimental therapeutic approaches. However, the instant disclosure provides BCI as a means to help triage those patients more appropriately.

In some embodiments, the calculated BCI indicates a low risk distant recurrence in the subject if treated with an aromatase inhibitor, targeted therapy or endocrine therapy, such as tamoxifen, during an initial period of 5 years or less because the calculated BCI indicates low risk or intermediate risk. The subject may thus be treated for the initial 5 year period with the aromatase inhibitor, targeted therapy or endocrine therapy with a low risk of recurrence. Following the initial period, and if the calculated BCI is of intermediate risk, the indication is that the subject is high-risk for cancer recurrence if not treated with an aromatase inhibitor, targeted therapy or endocrine therapy for an additional five year period.

In other embodiments, calculated BCI indicates a high risk distant recurrence in the subject if treated with an aromatase inhibitor, targeted therapy or endocrine therapy, such as tamoxifen, during an initial period of 5 years or less because the calculated BCI is of high risk or intermediate risk. The subject may thus be treated with the aromatase inhibitor, targeted therapy or endocrine therapy with an attenuated expectation of success. In some cases, the subject may be further treated with additional therapy, such as chemotherapy or radiation therapy as non-limiting examples, during the initial period.

The instant disclosure includes the identification of a subject as expected to benefit from additional therapy after recurrence-free survival during the course of an initial anti-aromatase, targeted therapy or endocrine therapy, such as for a period for five years or less.

Therefore, the disclosure includes determining the BCI as an indicator of increased likelihood of cancer recurrence in the subject following an initial anti-aromatase therapy, targeted therapy or endocrine therapy, such as adjuvant tamoxifen therapy. The methods may thus include identifying the subject as likely, or unlikely, to experience local or distant cancer recurrence. As a non-limiting example, determination of a likelihood of recurrence in the absence of an extended, post-initial treatment, therapy may be applied during a subsequent period for up to five years or more.

The present disclosure also provides for determining the risk of cancer recurrence in a breast cancer subject by performing individual risk assessment as part of, or in relation to, calculated BCI as a continuous variable. As disclosed herein, the determination of BCI in a population of breast cancer samples indicates that BCI is a continuous variable that correlates with risk of cancer recurrence in breast cancer afflicted subjects. Thus the risk of recurrence increases in a linear relationship with increasing BCI values. In some embodiments, the range of BCI values as a continuous variable is compared with a BCI value determined for an individual breast cancer sample to assess the risk of cancer recurrence as low-risk, intermediate-risk, or high-risk.

The additional or subsequent period of treatment or therapy as disclosed herein may occur anytime following the first-line (initial) therapy, such as immediately afterward, within three months after termination of first-line therapy, within six months after termination of first-line therapy, within nine months after termination of first-line therapy, within 12 months after termination of first-line therapy, within 18 months after termination of first-line therapy, or within 24 months (or more) after termination of first-line therapy.

The prognostic ability to identify high or low risk of recurrence provides information that can be instrumental in determining a course of treatment. For example, when BCI indicates that a subject (e.g., patient) is at a high risk of distant recurrence if treated with an aromatase inhibitor, targeted therapy or endocrine therapy, such as tamoxifen, during an initial period of 5 years or less, such therapies would be contraindicated, and other therapies, such as chemotherapy can be instituted.

Conversely, if BCI indicates that the subject is at low risk of distant recurrence if treated with an aromatase inhibitor, targeted therapy or endocrine therapy, such as tamoxifen, during an initial period of 5 years or less, treatment with an aromatase inhibitor, targeted therapy or endocrine therapy might be indicated.

Additionally, if BCI indicates that the subject is at high risk of distant recurrence if not treated with an aromatase inhibitor, targeted therapy or endocrine therapy during an additional 5 year period, treatment with an aromatase inhibitor, targeted therapy or endocrine therapy or another adjuvant therapy might be indicated.

In other embodiments, the subject is identified as having undergone treatment with an aromatase inhibitor, targeted therapy or endocrine therapy for a period of time up to five years without cancer recurrence. The subject is then classified, based on the BCI value of the subject's tumor, as having or not having a high-risk of distant recurrence of cancer after termination of the treatment. If the subject has a high risk of recurrence, the subject is then treated for an additional period with aromatase inhibitor, targeted therapy or endocrine therapy.

As is known, a high *HOXB13*/*IL17BR* index is not only prognostic, but also predictive of benefit of adjuvant endocrine treatment. See U.S. Patent 7,504,214 and PCT Patent Publication WO/2012/079059. As discussed in Example 2, the ability of BCI-C to predict responsiveness might be confounded by the dual prognostic and endocrine treatment predictive properties of *HOXB13*/*IL17BR.* By contrast, BCI-L contains only additive functions of MGI and *HOXB13*/*TL17BR* and was developed in an untreated group of breast cancer subjects in which clinical outcome represented the natural history of breast cancer. Findings reported in Example 2 suggest that BCI-L was spared any confounding effects of the endocrine treatment predictive properties of HOXB13/IL17BR, and as a result BCI-L is a preferred prognostic version of the combination of HOXB13/IL17BR and MGI.

In a second aspect, the disclosure provides a method that identifies responsiveness to treatment, for example an additional treatment with an aromatase inhibitor, targeted therapy or endocrine therapy after an initial treatment with an aromatase inhibitor, targeted therapy or endocrine therapy for five years or less. In some embodiments, the additional treatment is for a period of five years or more after the initial treatment period.

Thus, in further embodiments, the disclosure is directed to a method of determining need for extended treatment of a subject afflicted with breast cancer. The method comprises
determining mRNA expression levels of *HoxB13, IL17BR, Bub1B, CENPA, NEK2, RACGAP1,* and *RRM2* in a sample of ER+ breast cancer cells from the subject;
summing the expression levels to form a Breast Cancer Index (BCI) value, where a lower BCI value is correlated with not requiring any further additional treatment with an aromatase inhibitor, targeted therapy or endocrine therapy after an initial treatment with an aromatase inhibitor, targeted therapy or endocrine therapy for five years or less; and
classifying the sample, based on the BCI, as indicating the requirement or lack of requirement for additional treatment.

The ability of BCI to discern risk of recurrence and response to treatment can be combined to provide treatment options.

Thus, the disclosure is also directed to a method of treating a subject afflicted with breast cancer. The method comprises
determining mRNA expression levels of *HoxB13, IL17BR, Bub1B, CENPA, NEK2, RACGAP1,* and *RRM2* in a sample of ER+ breast cancer cells from the subject;
summing the expression levels to form a Breast Cancer Index (BCI) value, where a lower BCI value is correlated with (a) responsiveness to additional treatment with an aromatase inhibitor, targeted therapy or endocrine therapy after an initial treatment with an aromatase inhibitor, targeted therapy or endocrine therapy for five years or less and (b) a lower risk of distant recurrence; and
treating the subject consistent with the BCI value determination for the subject.

In some embodiments, BCI analysis indicates a high risk of distant recurrence in the subject if treated with an aromatase inhibitor, targeted therapy or endocrine therapy during an initial period of 5 years or less. Such a subject might be treated with chemotherapy.

In other embodiments, BCI analysis indicates a low risk of distant recurrence in the subject if treated with an aromatase inhibitor, targeted therapy or endocrine therapy for 5 years or less. Such a subject might be treated with the aromatase inhibitor, targeted therapy or endocrine therapy for the initial 5 year period, then not have to have extended therapy.

In additional embodiments, BCI analysis indicates a high risk of distant recurrence in the subject if not treated with an aromatase inhibitor, targeted therapy or endocrine therapy during an additional 5 year period. Such a subject might be treated with an aromatase inhibitor, targeted therapy or endocrine therapy or another adjuvant therapy. Any of the above methods can be useful for determining a prognostic factor or predictor of clinical responsiveness in pre-menopausal women and post-menopausal women. Post-menopausal women may be defined as those that are ≥50 years old while pre-menopausal women may be defined as those who are less than 50 years old. In some aspects, these women have undergone treatment with anti-aromatase, targeted therapy or endocrine therapy and remained cancer-free during that time.

In a further embodiment, the disclosure provides for the identification of the gene expression patterns by analyzing global, or near global, gene expression from single cells or homogenous cell populations that have been dissected away from, or otherwise isolated or purified from, contaminating cells beyond that possible by a simple biopsy. Because the expression of numerous genes fluctuate between cells from different patients as well as between cells from the same patient sample, the levels of gene expression may be determined in correspondence to one or more "control" or "normalization" genes, the expression(s) of which are relatively constant in the cells of a patient or between patients.

One advantage of this approach is that contaminating, non-cancer cells (such as infiltrating lymphocytes or other immune system cells) are not present to possibly affect the genes identified or the subsequent analysis of gene expression to identify the cancer recurrence and/or survival outcomes of patients. Such contamination is present where a biopsy containing many cell types is used to assay gene expression profiles.

While the present disclosure is described mainly in the context of human cancer, such as breast cancer, it may be practiced in the context of cancer of any animal. Preferred animals for the application of the present disclosure are mammals, particularly those important to agricultural applications (such as, but not limited to, cattle, sheep, horses, and other "farm animals"), animal models of cancer, and animals for human companionship (such as, but not limited to, dogs and cats).

The methods provided by the disclosure may also be automated in whole or in part. Kits

The materials for use in the methods of the present disclosure are ideally suited for preparation of kits produced in accordance with well-known procedures. The disclosure thus provides kits comprising agents for the detection of expression of the disclosed genes for grading tumors or determining cancer outcomes. Such kits optionally comprise the agent with an identifying description or label or instructions relating to their use in the methods of the present disclosure. Such a kit may comprise containers, each with one or more of the various reagents (typically in concentrated form) utilized in the methods, including, for example, prefabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more primer complexes of the present disclosure (e.g., appropriate length poly(T) or random primers linked to a promoter reactive with the RNA polymerase). A set of instructions is also typically included.

Preferred embodiments are described in the following examples. Other embodiments within the scope of the claims herein will be apparent to one skilled in the art from consideration of the specification or practice of the invention as disclosed herein.

### Example 1

### Study design and patients

For a prospective comparison study, tissue samples were obtained from the TransATAC project, initiated in 2002 to establish a tissue bank of formalin-fixed paraffin-embedded (FFPE) primary tumor blocks from postmenopausal patients with estrogen-receptor-positive breast cancer from the mono therapy groups of the ATAC trial to assist with translational research (Paik et al., 2004; Dowsett et al., 2010). Archival tumor blocks were requested for all patients for whom the 21-gene recurrence score and IHC4 had already been calculated, except those known to be estrogen-receptor and progesterone-receptor negative according to local tests and those randomly assigned to the combination treatment group of the ATAC trial. The study was approved by the South-East London Research Ethics Committee and the Massachusetts General Hospital Institutional Review Board. Patients had provided written consent for their tissue to be used in further trials.

### Procedures

Previously, a study was done in which RNA was extracted from FFPE blocks from the TransATAC tissue bank from UK patients (whose samples made up 79% of the collection) to calculate and test the 21-gene recurrence score (Paik et al., 2004). Subsequently, immunohistochemical analysis for estrogen receptors, progesterone receptors, HER2, Ki-67 and tumor grade assessment were undertaken, and IHC4 and clinical treatment score (a prognostic model using the classic variables of tumor size and grade, lymph node status, age, and treatment) were calculated using tissue samples from the same patients whose tissue was used to calculate the 21-gene recurrence score (for whom sufficient additional tissue was available).

In this study, the same matched samples as used in the previous studies with sufficient residual RNA were used to undertake BCI analysis. The genes were tested, the primer and probe sequences were analyzed, and RT-PCR procedures were performed to calculate *HOXB13*/*IL17BR* and MGI as previously reported (US Patents 7,930,105 and 7,504,214, US Patent Publications 2011/0136680 and 2013/0281502, and PCT Patent Publication WO/2012/079059). Two prespecified BCI models were tested. The models were cubic (BCI-C) and linear (BCI-L), based on cubic and linear combinations of the variables.

The BCI score was linearly scaled to a final score (0-10). Groups were identified as low-risk, intermediate-risk, and high-risk with prespecified cutoff points for each model: BCI-C low risk (<5.0 points), BCI-C intermediate risk (5.0 to 6.4), and BCI-C high risk (>6.4); and BCI-L low risk (<5.0825 points), BCI-L intermediate risk (5.0825 to 6.5025), and BCI-L high risk (>6.5025). The 21-gene recurrence core risk groups were identified as previously reported (Paik et al., 2004). Three IHC4 risk groups were established using two cutoff points that corresponded to a 10 year distant recurrence rate of 10% and 20% (i.e., <10%, ≥10% to 20%, and >20%) in the TransATAC cohort, respectively. The IHC4 cutoffs have not been independently validated.

Distant recurrence was prospectively defined as the primary endpoint, which refers to all recurrences at distant organs, excluding contralateral disease, locoregional and ipsilateral recurrences, and other second primary cancers. Also included were distant recurrence that took place after locoregional recurrence as an event at the time of distant recurrence. Patients who died before distant recurrence were excluded. All recurrences, breast cancer deaths, and overall survival (time to death from any cause) were defined as secondary endpoints. The primary analysis population was patients with estrogen receptor-positive, NO breast cancer, whereas the secondary analysis populations included patients with estrogen receptor-positive, NO, HER2-negative breast cancer and those with estrogen receptor-positive, node positive breast cancer. The primary study objective was prospectively defined as assessment of overall (0 to 10 year) prognostic ability of the BCI-C model for distant recurrence in patients with estrogen-receptor-positive, NO breast cancer. Secondary objectives were to assess the prognostic ability of the BCI-L model and its component, *HOXB13*/*IL17BR* and MGI, for overall (0 to 10 year), early (0 to 5 year), and late (5 to 10 year) distant recurrence, as well as to compare the ability of BCI-L with that of the recurrence score and IHC4.

### Statistical analysis

A statistical analysis plan was approved by the steering committee for the ATAC and LATIE (Long-term Anastrozole versus Tamoxifen Treatment Effects) trials before study initiation. Early distant recurrences were assessed by censoring follow-up of all patients 5 years after diagnosis. Late distant recurrences were assessed within the subset of patients who remained distant recurrence free for at least 5 years to assess whether the gene signature remained prognostic after its prognostic effect for early recurrence was removed. Likelihood ratio tests based on Cox proportional hazards regression models were used to test for a significant difference between a reduced proportional hazards model based on clinical treatment score and a full proportional hazards model, including BCI, 21-gene recurrence core, or IHC4. The improvement in prediction was quantified by the change in the likelihood ratio χ² (LR-Δχ²) value, which measures the amount of information added to the proportional hazards model by tile gene signatures compared with clinical treatment score. Because IHC4 was developed in a subset of TransATAC samples, sample splitting was done, as previously described, to adjust for potential overfilling. Kaplan- Meier survival analysis was used to graphically present the proportion of patients with distant recurrence in BCI's three prespecified risk groups, and tested the quality of the curves with a log-rank test.

The risk of distant recurrence was calculated as a function of BCI as a linear covariate from Cox proportional hazards models for overall (0-10 years), early (0-5 years), and late (5-10 years) distant recurrence. To compare BCI, 21-gene recurrence score, and IHC4, the interquartile hazard ratio (HR) was estimated by comparing the 75th percentile versus the 25th percentile of the continuous scores of the biomarkers and the associated 95% CI from Cox proportional hazards models. A two- sided p value of less than 0.05 was regarded to be statistically significant. Because the recurrence score had already been studied in TransATAC, and IHC4 was developed in a subset of these patients, the ability of the 21-gene recurrence score and IHC4 as continuous scores was prespecified, and there was no performance of any multiple testing adjustment. Statistical analyses were performed with STATA version 12.1.

### Example 2

### Patients and samples

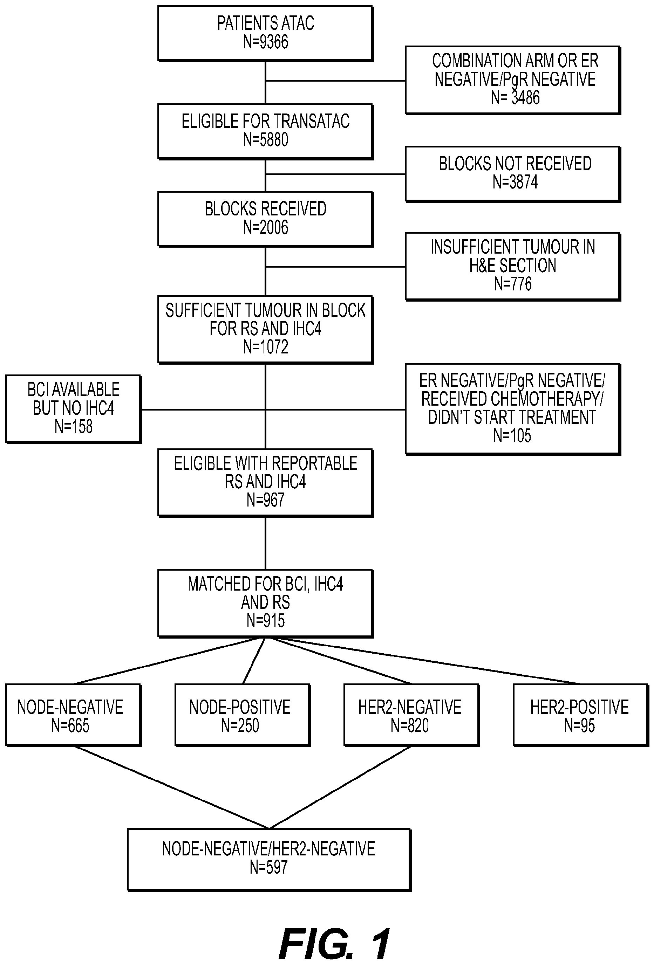

Values using the 21-gene recurrence score, IHC4, and BCI were calculated for 915 women, of whom 665 had estrogen-receptor-positive, NO breast cancer (FIG 1). Clinical characteristics of these 665 patients are listed in Table 2 and compared with the characteristics of 561 UK patients with estrogen- receptor-positive, NO breast cancer who participated in the ATAC trial but who were not part of TransATAC. No significant difference between these two groups, except that the non-TransATAC cohort had significantly more well-differentiated tumors and less moderately differentiated tumors than the TransATAC patients, and significantly fewer late distant recurrences.

**Table 1. Patient demographic and clinical characteristics**

| | N0 BCI cohort | N0 HER2neg | N0 UK patients | |
|---|---|---|---|---|
| | TransATAC | BCI cohort | Non-TransATAC* | |
| | (n=665) | TransATAC (n=597) | (n=561) | P value# |
| Age, mean (SD) | 63.3 (8.1) | 63.4 (8.0) | 62.6 (7.8) | 0.12 |
| BMI, mean (SD) | 27.1 (4.8) | 27.2 (4.8) | 26.8 (5.1) | 0.28 |
| Tumor size | | | | 0.13 |
| <2cm | 486 (73.1%) | 442 (74.1%) | 432 (77.0%) | |
| 2-3 cm | 144 (21.7%) | 125 (20.9%) | 95 (16.9%) | |
| >3cm | 35 (5.2%) | 30 (5%) | 29 (5.2%) | |
| Unknown | 0 | 0 | 5 (0.9%) | |
| Tumor grade | | | | 0.0051 |
| Well | 143 (21.5%) | 138 (23.1%) | 155 (27.6%) | |
| Moderate | 395 (59.4%) | 357 (59.8%) | 300 (53.5%) | |
| Poor | 127(19.1%) | 102(17.1%) | 78 (13.9%) | |
| Unknown | 0 | 0 | 28 (5.0%) | |
| Radiotherapy | | | | 0.95 |
| No | 220 (33.1%) | 189(31.7%) | 187 (33.3%) | |
| Yes | 445(66.9%) | 408 (68.3%) | 374 (66.7%) | |
| Mastectomy | | | | 0.86 |
| No | 439 (66.0%) | 404 (67.7%) | 374 (66.7%) | |
| Yes | 226 (34.0%) | 193 (32.3%) | 187 (33.3%) | |
| Treatment | | | | |
| Anastrozole | 337 (50.7%) | 309 (51.8%) | 285 (50.8%) | 0.95 |
| Tamoxifen | 328 (49.3%) | 288 (48.2%) | 276 (49.2%) | |
| Distant Recurrence | | | | |
| Early (0-5 years) | 33 (5.0%) | 21 (3.5%) | 23 (4.1%) | 0.56 |
| Late (5-10 years) | 39 (5.9%) | 36 (6.6%) | 12 (2.3%) | 0.0022 |

| | | | | |
|---|---|---|---|---|
| ^{∗}these are patients from the United Kingdom in the ATAC trial who do not have tumor blocks available for the translational study. #comparison is between NO TransATAC versus NO Non-TransATAC cohorts, t tests were used for age and BMI, proportional test based on normal approximation was used for distant recurrence, all others used Fisher's exact test. Abbreviations: ER, estrogen receptor; NO, node negative; HER2neg, human epidermal growth factor receptor 2 negative; BMI, body mass index; UK, United Kingdom | | | | |

In NO women in the BCI TransATAC cohort, there were 106 recurrences, including 72 distant recurrences and seven local recurrences after mastectomy. Median follow-up in the BCI TransATAC cohort was 9.97 years (IQR 8.5 to 10).

### Calculation of H/I and MGI

Generally, and with respect to MGI, it is preferred that the expression levels of the disclosed genes are combined to form a single index that serves as a strong prognostic factor and predictor of clinical outcome(s). The index is a summation of the expression levels of the genes used and uses coefficients determined from principal component analysis (PCA) to combine cases of more than one disclosed gene into a single index. The coefficients are determined by factors such as the standard deviation of each gene's expression levels across a representative dataset, and the expression value for each gene in each sample. The representative dataset is quality controlled based upon the average expression values for reference gene(s) as disclosed herein.

Stated differently, normalized expression levels for the five genes from, e.g., microarrays, next-generation sequencing or RT-PCR were standardized to a mean of 0 and standard deviation of 1 across samples within each dataset and then combined into a single index per sample via PCA using the first principal component. Standardization of the primary expression data within each dataset was necessary to account for the different platforms (microarrays, sequencing and rtPCR) and sample types (frozen and FFPE). As a result, and following scaling parameters, a formula for the summation of expression values that defines the index is generated. The precision of the scaling parameters can then be tested based on the means, standard errors, and standard deviations (with confidence intervals) of the expression levels of the genes across the data set. Therefore, generation of the formula for the index is dependent upon the dataset, reference gene, and genes of the MGI.

The *HOXB13:IL17BR* ratio was calculated as the difference in standardized expression levels between *HOXB13* and *IL17BR* as described previously (Ma et al., 2006). The means and standard deviations for *HOXB13* and *IL17BR* used for standardizing the Table 2 cohort may be derived from an analysis of 190 FFPE tissue sections from a separate population-based cohort of estrogen receptor-positive, lymph node-negative breast cancer patients.

For MGI, obviously abnormal raw CT values were removed prior to averaging the values over duplicates for each gene and each sample. The averaged raw CT value for each gene was then normalized by the averaged CT value of four reference genes (ACTB, HMBS, SDHA, and UBC). The normalized expression levels (CT) compared to a pre-determined cutoff value, such as 0, where high MGI is above the cutoff and low MGI is below the cutoff.

### Breast Cancer Index (BCI)

BCI is built by combining H:I and MGI as continuous variables. The linearity of these two variables were checked by fitting a Cox proportional hazard regression model with restricted cubic splines, and H:I demonstrated significant non-linearity. A polynomial function of H:I was used to approximate the restricted models using Akaike information criterion. The resulting predictor from the final Cox regression model was then re-scaled into the range of 0 to 10, which is referred to as the BCI.

The BCI is further categorized into three levels: low risk, intermediate risk, and high risk as described herein.

H/I CUT-POINT: The cut-point of 0.06 for the *HOXB13:IL17BR* ratio, previously defined to stratify patients treated with adjuvant tamoxifen into low and high risk of recurrence, was used in this study.

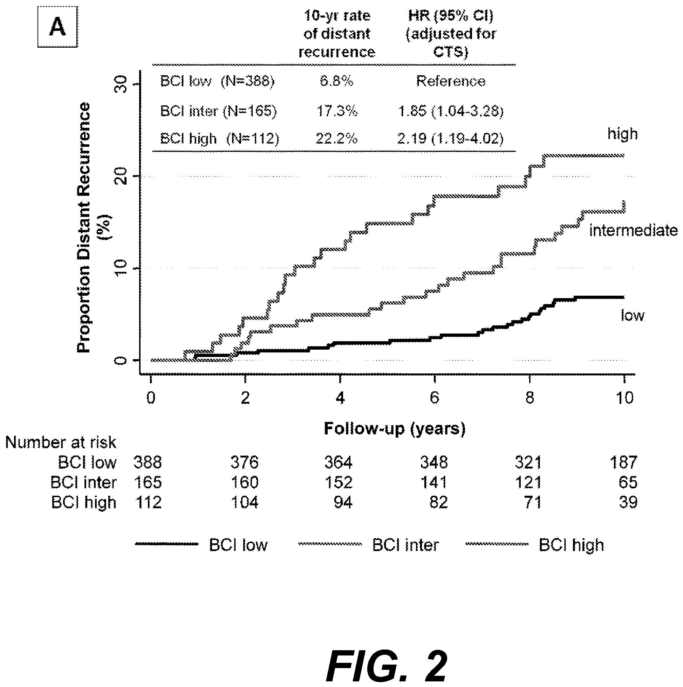

In the 665 estrogen-receptor-positive, NO patients, Kaplan-Meier analysis of the BCI-C model showed significant differences in absolute distant recurrence over a 10 year period (p<0.001) in the prespecified categorical BCI-C risk groups, and differences in the HRs between the low-risk group and the other risk groups, after adjustment for the effects of tumor size and grade, age, and treatment (as determined by clinical treatment score; see FIG. 2A). BCI-C analyzed as a continuous variable, rather than as subgroups with defined cutoffs, was not significantly associated with overall (0 to 10 year) risk of distant recurrence when adjusted for clinical treatment score (interquartile HR 1.39; LR-Δχ²=3.70; p=0.054).

Assessment of BCI-L in the same population of patients showed that this version was much more strongly associated with overall risk of distant recurrence than was BCI-C when adjusted for clinical treatment score (interquartile HR 2.30; LR-Δχ² =22.69; p<0.0001: Table 2).

**Table 2.**

| | **All recurrence (0-10 years)** | | **Early recurrence** (**0-5 years**) | | **Late recurrence** (**5-10 years**) | |
|---|---|---|---|---|---|---|
| | **HR^{X}** (**95%CI**) | **LR-Δχ²** (**p value**) | **HR^{X}** (**95%CI**) | **LR-Δχ²** (**p value**) | **HR^{∗}** (**95%CI**) | **LR-Δ χ²** (**p value**) |
| **Univariate** | | | | | | |
| BCI | | | | | | |
| N0 | 3.12(2.25-4.32) | 49.07(p<0.0001) | 4.11(2.52-6.70) | 34.58(p<0.0001) | 2.47(1.59-3.83) | 17.37(p<0.0001) |
| N0 HER2-negative | 3.30(2.30-4.73) | 46.03(p<0.0001) | 4.22(2.32-7.64) | 25.86(p<0.0001) | 2.84(1.80-4.48) | 22.66(p<0.0001 ) |

| 21- gene recurrence score | | | | | | |
|---|---|---|---|---|---|---|
| N0 | 1.64(1.39-1.94) | 27.37(p<0.0001) | 1.96(1.60-2.41) | 28.09(p<0.0001) | 1.28(0.95-1.72) | 2.99(p=0.21) |
| NO HER2-negative | 1.89(1.45-2.47) | 19.55(p<0.0001) | 2.38(1.61-3.53) | 16.18(p<0.0001) | 1.59(1.09-2.31) | 6.65(p=0.014) |
| IHC4 | | | | | | |
| N0 | 2.30(1.80-2.95) | 40.90(p<0.0001) | 3.38(2.39-4.78) | 42.46(p<0.0001) | 1.55(1.06-2.26) | 5.58(p=0.022) |
| NO HER2-negative | 2.66(1.85-3.81) | 27.04(p<0.0001) | 4.08(2.26-7.36) | 22.13(p<0.0001) | 2.06(1.29-3.28) | 9.32(p=0.0034) |

| **Multivariate including clinical treatment score** | | | | | | |
|---|---|---|---|---|---|---|
| BCI | | | | | | |
| N0 | 2.30(1.62-3.27) | 22.69(p<0.0001) | 2.77(1.63-4.70) | 15.42(p<0.0001) | 1.95(1.22-3.14) | 7.97(p=0.0048) |
| N0 HER2-negative | 2.49(1.68-3.68) | 21.99(p<0.0001) | 3.26(1.96-6.30) | 13.65(p=0.00023) | 2.12(1.30-3.47) | 9.453(p=0.0021) |

| **21- gene recurrence score** | | | | | | |
|---|---|---|---|---|---|---|
| NO | 1.48(1.22-1.78) | 13.68(p=0.0002) | 1.80(1.42-2.29) | 18.48(p<0.0001) | 1.13(0.82-1.56) | 0.48(p=0.47) |
| NO HER2-negative | 1.52(2.15-2.02) | 7.65(p=0.0055) | 1.93(1.26-2.96) | 8.37(p=0.0041) | 1.28(0.87-1.88) | 1.33(p=0.28) |
| IHC4 | | | | | | |
| N0 | 1.69(1.51-2.56) | 22.83(p=0.0001) | 2.90(2.01-4.18) | 29.14(p<0.0001) | 1.30(0.88-1.94) | 1.59(p=0.20) |
| N0 HER2-negative | 2.13(1.45-3.14) | 13.75(p=0.0002) | 3.41(1.83-6.39) | 13.83(p<0.0001) | 1.61(0.98-2.66) | 3.30(p=0.086) |

| | | | | | | |
|---|---|---|---|---|---|---|
| HR=hazard ratio, LR-Δχ² = change in the χ² value based on the likelihood ratio statistic. BCI = breast-cancer-index assay. NO = node negative, IHC4 = four immunohistochemical markers (estrogen receptor, progesterone receptor, HER2, and Ki-67). ^{∗}HR was calculated as between the IQR of the continuous scores of each biomarker; sample splitting was used to calculate HRs and χ² for IHC4. | | | | | | |

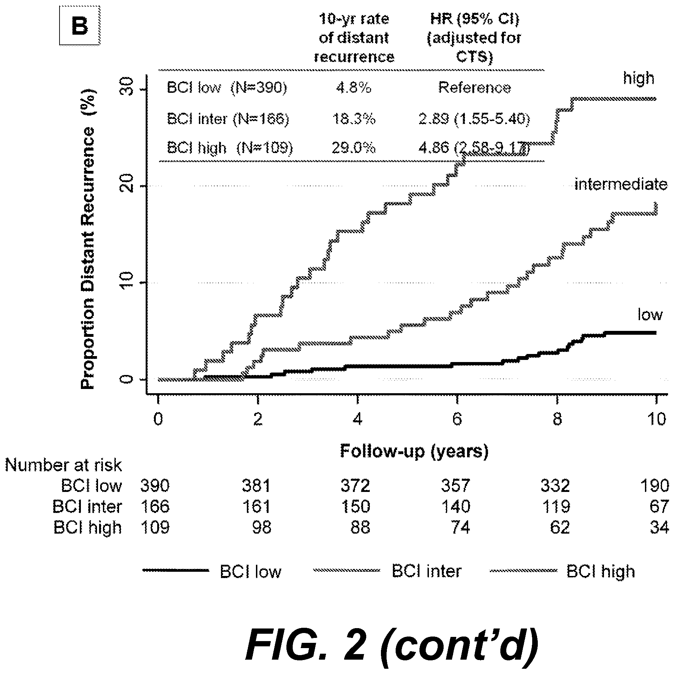

Kaplan-Meier curves show clear differences in absolute distant recurrence rates according to prespecified BCI-L risk groups (p<0.001; FIG. 2B). The overall 10-year risk of distant recurrence increased linearly with increasing BCI-L (FIG. 3).

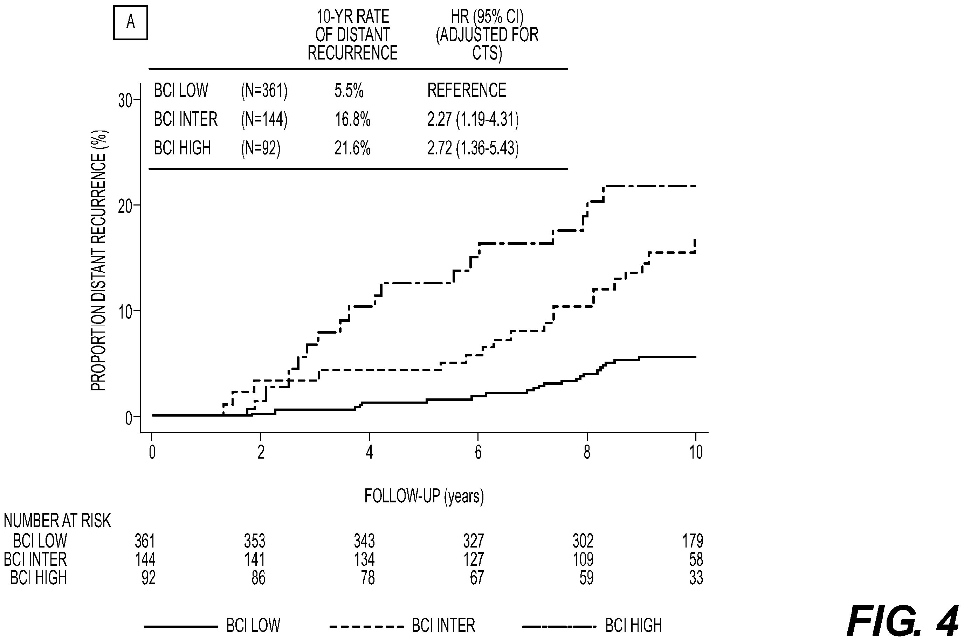

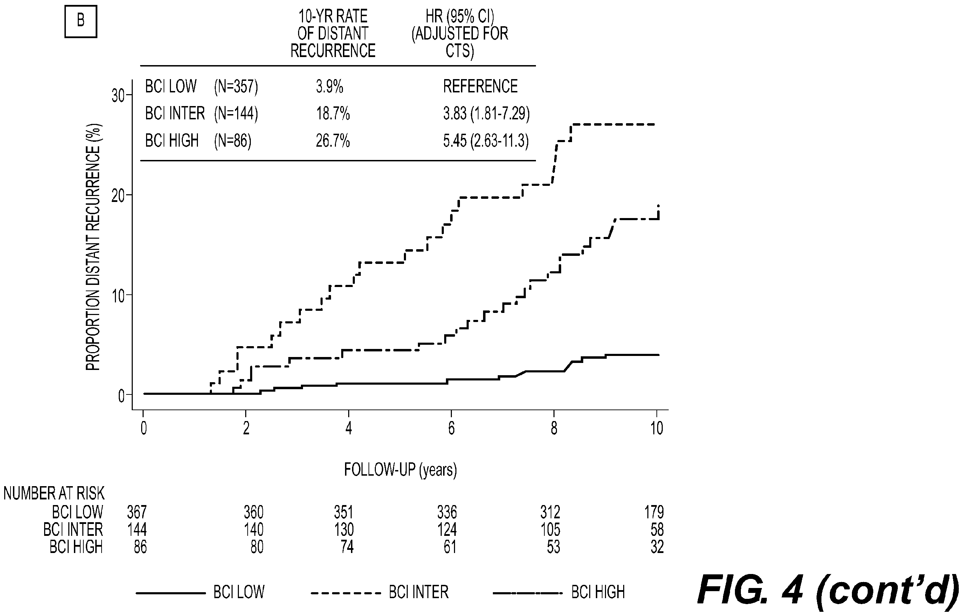

In the HER2-negative, NO subset of 597 patients, both BCI-C and BCI-L were significantly associated with overall risk of distant recurrence (BCI-C interquartile HR 1.65, LR-Δχ²=6.61, p=0.0001; BCI-L interquartile HR 2.49, LR-Δχ²=21.9, p<0.0001; Table 2). Kaplan-Meier curves of the prespecified groups for both versions of BCI showed distinct differences in absolute distant recurrence (FIG. 4A - BCI-C; 4B - BCI-L).

Comparison of the prognostic ability of BCI-L with that of BCI-C showed that, unlike BCI-C, BCI-L was a significant predictor of risk of recurrence as both a continuous and categorical variable, and the HR, after adjustment for clinical treatment score, was 2.19 versus 4.86 between high-risk and low-risk groups for BCI-C and BCI-L, respectively. Subsequent discussion below uses the linear model (referred to as BCI therein).

### Groups based upon BCI

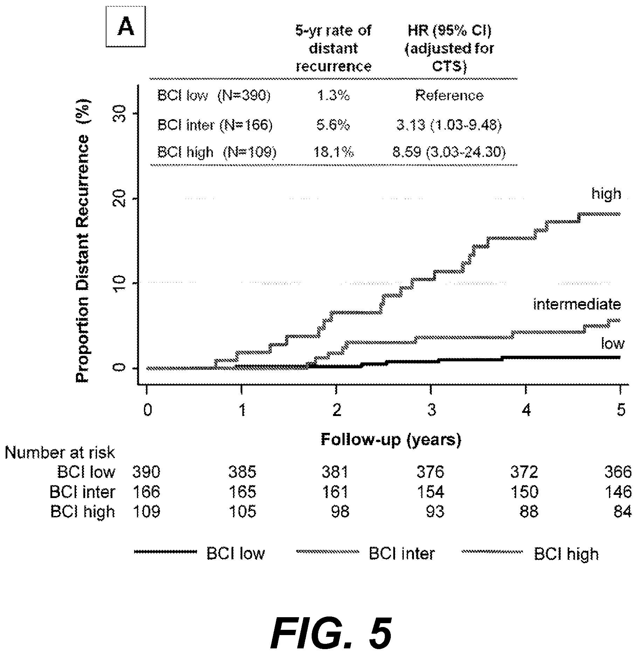

BCI was significantly associated with risk of early (0-5 year) distant recurrence (Table 3) when adjusted for clinical treatment score. Kaplan-Meier curves (FIG. 5A) displayed differences in absolute distant recurrence rate at 5 years. Although three risk groups were prespecified, the results from the prespecified Kaplan-Meier analysis showed low-risk and intermediate risk patient had similar rates of distant recurrence and constitute one group that is distinctly different from the group of high-risk patients.

A post-hoc Kaplan-Meier analysis showed little difference in distant recurrence at 5 years between the BCI low-risk and intermediate-risk groups, which contained 556 (84%) of 665 patients (PI) with a combined 5-year rate of distant recurrence of 2-6%; (Table 3).

**Table 3. Absolute risk of early and late distant recurrence in clinically relevant subsets of ER+ NO patients.**

| **Early Recurrence (0-5 Years)** | | |
|---|---|---|
| **Risk Subsets** | **N(%)** | **Risk of Early DR at 5 Years (95% CI)** |
| P1 (BCI low & intermediate risk) | 556 (84%) | 2.6% (1.5% - 4.3%) |
| P2 (BCI high risk) | 109 (16%) | 18.1% (12.0% -27.0%) |

| **Late Recurrence (5-10 Years)** | | |
|---|---|---|
| **Risk Subsets** | **N** (%) | **Risk of Early DR at 5 Years (95% CI)** |
| P3 (BCI low risk) | 366 (61%) | 3.5% (2.0% - 6.1%) |
| P4 (BCI intermediate & high risk) | 230 (39%) | 13.4% (9.3% - 19.0%) |

The BCI high-risk group (P2) that contained 109 (16%) of 665 patients, had a 5-year rate of distant recurrence of 18.1%. When adjusted for clinical treatment score, the HR between P1 and P2 was 4.61.

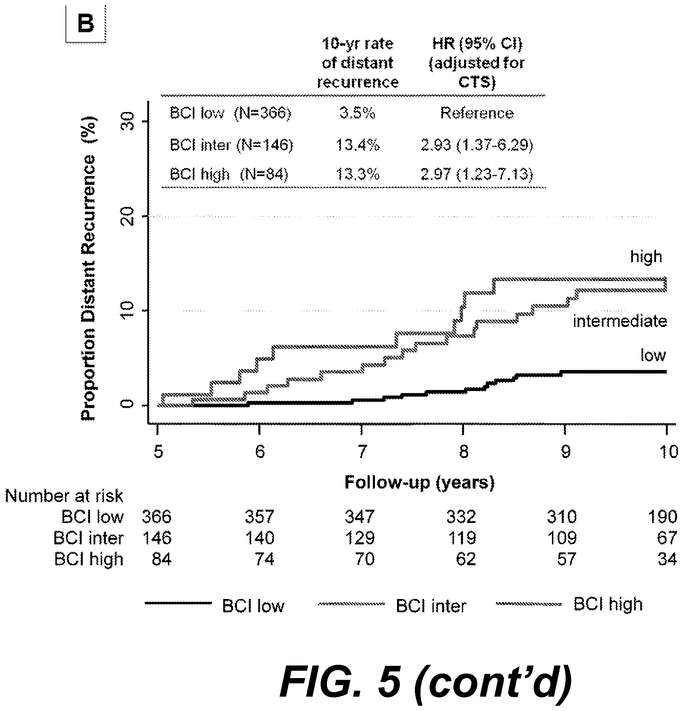

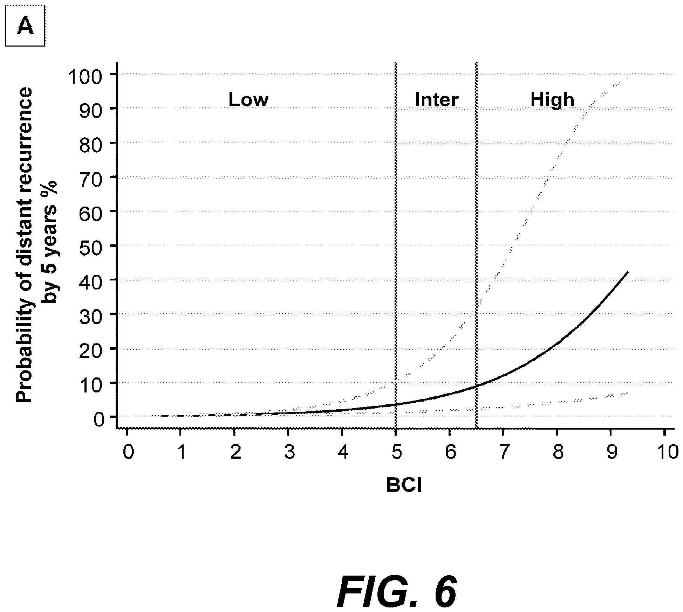

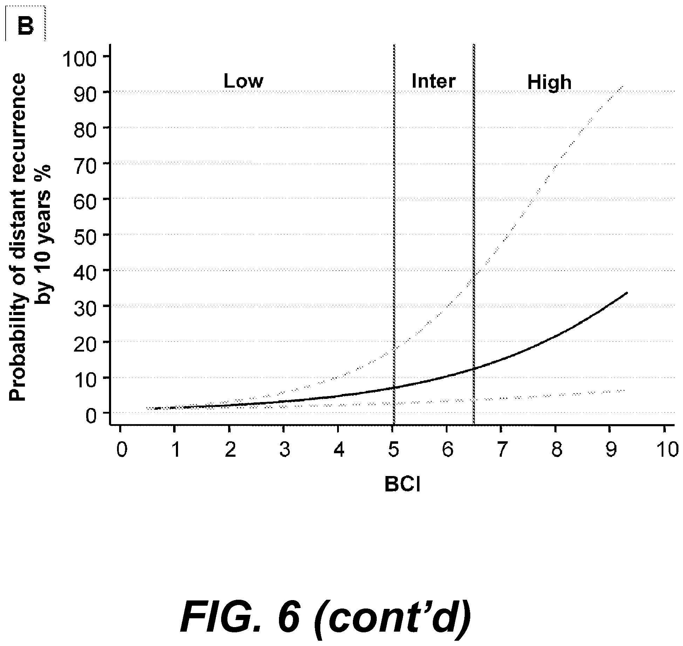

For late (5-10 year) recurrence, BCI was significantly associated with risk of distant recurrence when adjusted for clinical treatment score (Table 2). Kaplan-Meier curves showed differences in absolute distant recurrence rates for years 5-10 for the BCI low-risk, intermediate-risk, and high-risk groups (FIG. 5B). The results from the prespecified Kaplan-Meier analysis showed that intermediate-risk and high-risk patients had highly similar rates of recurrence, constituting one population that was distinctly different from the population of low-risk patients. Additional post-hoc Kaplan-Meier analyses (Table 3) showed the BCI low-risk group (P3) having distant recurrence rate of 3.5% for years 5-10, substantially different from the combined BCI intermediate-risk and high-risk groups (P4) rate of 13.4%). Adjusting for clinical treatment score, the HR between P3 and P4 was 2.94. The risk of distant recurrence increased linearly with increasing BCI values for both early and late recurrence (FIGS. 6A and 6B).

### HER2 status

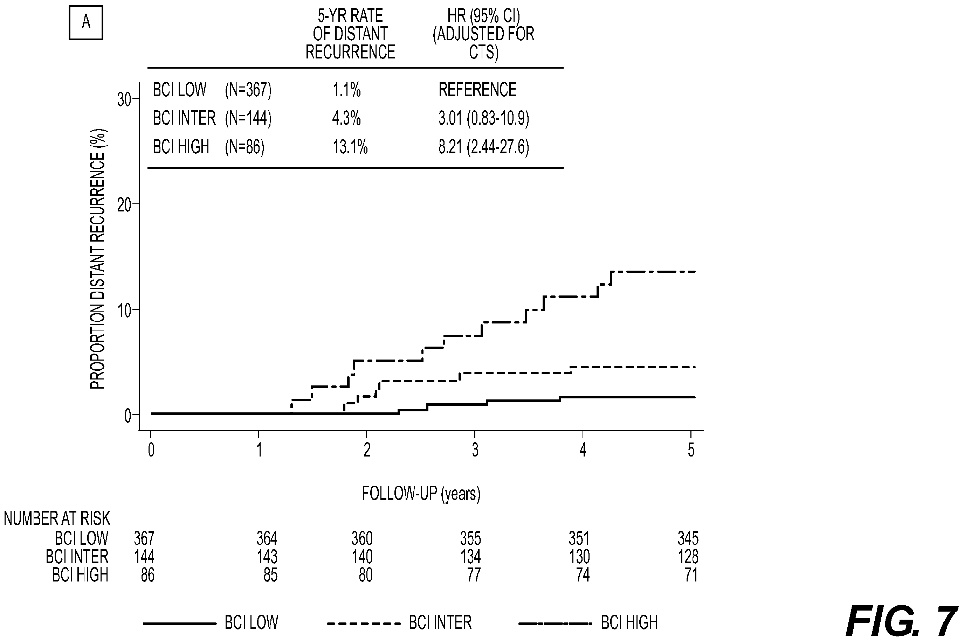

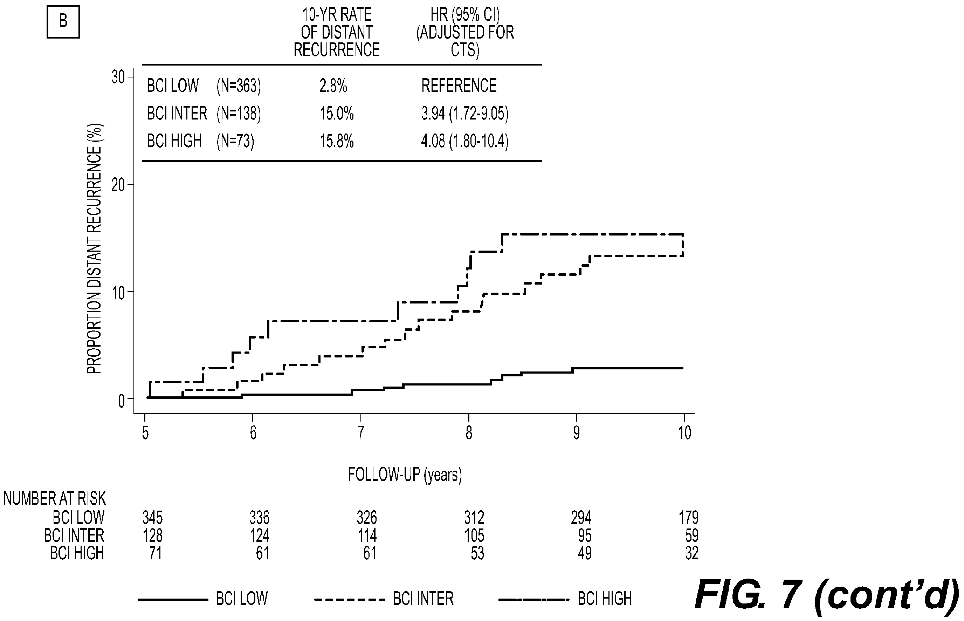

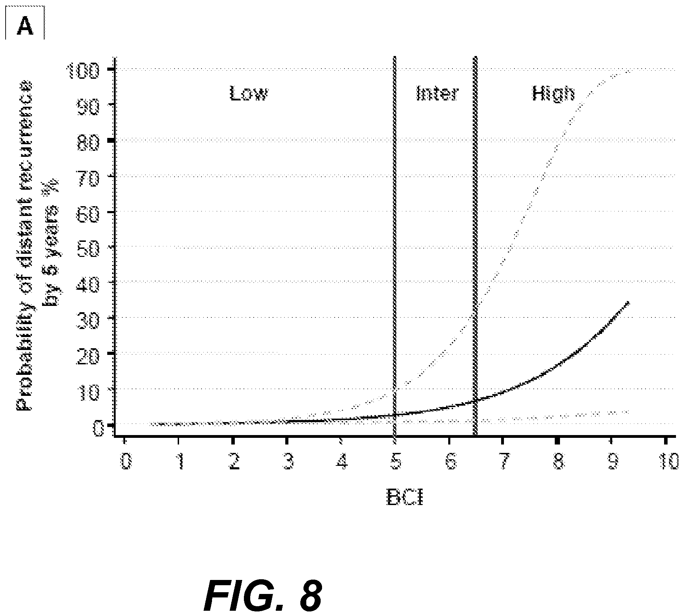

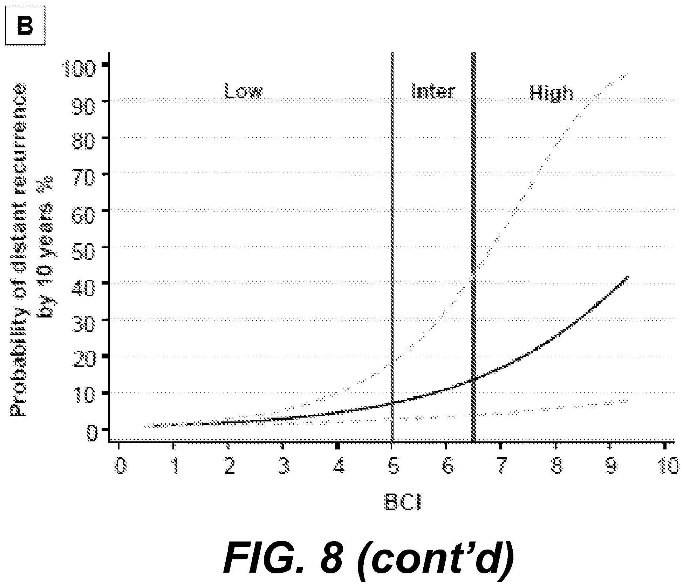

Because the natural history of estrogen-receptor-positive, HER2-positive breast cancer differs from that of estrogen-receptor-positive, HER2-negative breast cancer, a subset analysis was conducted to assess whether the prognostic ability of BCI in the entire NO estrogen-receptor-positive TransATAC cohort was unduly affected by the inclusion of the subset of HER2-positive patients. In the HER2-negative NO subset of 597 patient (90% of the total tested study group), BCI was significantly associated with risk of early distant recurrence and late distant recurrence (Table 2), as well as distinct differences in absolute distant recurrence according to BCI risk group (FIG. 7). For both early and late recurrence the risk of distant recurrence increased with increasing BCI values (FIG. 8).

### Aromatase inhibitors and endocrine therapy

Kaplan-Meier curves of overall (0-10 year) distant recurrence for 21-gene recurrence core and IHC4 risk groups for all patients, and separately according to treatment group (anastrozole or tamoxifen), are shown in FIG. 9. For all patients combined (i.e., those who received either anastrozole or tamoxifen), the BCI low-risk group had the lowest proportion of patients with distant recurrence in 10 years (4.8%) when compared with the 21-gene recurrence score low-risk group (6.5%) and the IHC4 low-risk group (6.2%), whereas the BCI high-risk group had the highest proportion of distant recurrence (29.0%) compared with the 21-gene recurrence score high-risk group (27.1%) and IHC4 high-risk group (21.8%; FIG. 9).

Additionally, as shown in FIGS. 9D-9F, BCI stratified the distant recurrence risk between the high and low risk anastrozole groups much better than the 21-gene and the IHC4 systems, since BCI had the highest % recurrence in its high risk group (21.6% vs. 13.5% and 15.6%), and also had the lowest % recurrence in its low risk group (4.8% vs. 9.4% and 8.0%).

### Comparison to other assessments

The change in likelihood ratio LR-Δχ² values was used to provide a direct head-to- head comparison of BCI with the IHC4 and the 21-gene recurrence score. The relative prognostic ability of each biomarker varied depending on the distant recurrence timeframe (Table 2). For early recurrence, BCI, IHC4, and the 21-gene recurrence score were all prognostic for distant recurrence in both univariate and multivariate analyses (Table 2). In all NO patients, IHC4 was more prognostic than recurrence score and BCI after adjusting for clinical treatment score. However, in the NO HER2-negative patients, BCI and IHC4 had similar prognostic abilities that were both better than that of the 21-gene recurrence score after adjusting for clinical treatment score (Table 2). In the multivariate analysis of late recurrence, only BCI remained strongly prognostic in all NO and NO HER2-negative patients, whereas both IHC4 and 21-gene recurrence score were not prognostic in either population (Table 2). Similar results were noted considering all recurrences, breast-cancer deaths, and overall survival as endpoints (Table 4).

**Table 4. Comparative prognostic performance for secondary early and late disease events of BCI, RS (21-gene recurrence score), and IHC4 in all hormone receptor-positive NO patients and the NO HER2- subset.**

| | | **Early Recurrence (0-5 Years)** | | **Late recurrence (5-10 Years)** | |
|---|---|---|---|---|---|
| | | **HR (95% CI)^{∗}** | **LR-Δχ² (P-value)** | **HR (95% CI)^{∗}** | **LR-Δ-χ2 (P-value)** |
| **UNIVARIATE** | | | | | |
| BCI | | ***All recurrences*** | | | |
| | N0 | 2.58 (1.74-3.82) | 23.08 (<0.0001) | 1.79 (1.26-2.54) | 10.53 (0.0012) |
| | N0/HER2- | 2.20 (1.40-3.45) | 11.92 (0.00061) | 1.95 (1.37-2.79) | 13.60 (0.00021) |
| RS | N0 | 1.90 (1.57-2.29) | 32.11 (<0.0001) | 1.28 (0.95-1.72) | 2.02 (0.15) |
| | N0/HER2- | 2.03 (1.46-2.82) | 14.62 (0.00012) | 1.48 (1.09-2.02) | 5.52 (0.018) |
| IHC4 | N0 | 2.52 (1.88-3.40) | 33.37 (<0.0001) | 1.55 (1.06-2.26) | 4.47 (0.034) |
| | N0/HER2- | 2.48 (1.54-3.98) | 13.23 (0.00031) | 1.91 (1.31-2.78) | 10.50 (0.0012) |
| BCI | | ***Breast cancer death*** | | | |
| | N0 | 5.82 (3.10-10.92) | 34.36 (<0.0001) | 2.23 (1.29-3.86) | 8.42 (0.0037) |
| | N0/HER2- | 7.30 (3.22-16.51) | 26.42 (<0.0001) | 2.52 (1.42-4.45) | 10.38 (0.0013) |
| RS | N0 | 2.05 (1.62-2.60) | 24.15 (<0.0001) | 1.41 (1.01-1.99) | 3.24 (0.072) |
| | N0/HER2- | 2.90 (1.80-4.65) | 15.35 (0.0001) | 1.78 (1.14-2.77) | 5.36 (0.021) |
| | N0 | 3.66 (2.40-5.56) | 33.84 (<0.0001) | 1.73 (1.09-2.75) | 5.03 (0.024) |
| IHC4 | N0/HER2- | 4.91 (2.32-10.41) | 16.62 (<0.0001) | 2.26 (1.27-4.01) | 7.13 (0.0076) |
| | | ***Overall survival*** | | | |
| BCI | N0 | 2.25 (1.54-3.28) | 18.26 (<0.0001) | 1.96 (1.43-2.68) | 17.87 (<0.0001) |
| | N0/HER2- | 2.04 (1.34-3.12) | 11.07 (0.00091) | 2.04 (1.47-2.82) | 18.63 (<0.0001) |
| RS | N0 | 1.54 (1.26-1.89) | 13.59 (0.00020) | 1.19 (0.94-1.50) | 1.93 (0.16) |
| | N0/HER2- | 1.58 (1.12-2.24) | 5.86 (0.016) | 1.33 (0.99-1-78) | 3.17 (0.075) |
| IHC4 | N0 | 1.84 (1.37-2.48) | 14.45(0.00010) | 1.25 (0.94-1.67) | 2.21 (0.14) |
| | N0/HER2- | 1.57 (0.98-2.51) | 3.36(0.066) | 1.45 (1.01-2.07) | 3.97 (0.046) |

| **MULTIVARIATE INCLUDING CTS** | | | | | |
|---|---|---|---|---|---|
| BCI | | ***All recurrences*** | | | |
| | N0 | 1.99 (1.27-2.99) | 9.71 (0.0018) | 1.49 (1.02-2.17) | 4.25 (0.039) |
| | N0/HER2- | 1.83 (1.12-3.01) | 5.93 (0.014) | 1.57 (1.07-2.30) | 5.34 (0.021) |
| RS | N0 | 1.76 (1.43-2.17) | 22.21 (<0.0001) | 1.10 (0.83-1.46) | 0.44 (0.51) |
| | N0/HER2- | 1.80 (1.26-2.56) | 9.41 (0.0022) | 1.27 (0.92-1.74) | 2.01 (0.16) |
| IHC4 | N0 | 2.22 (1.62-3.03) | 22.48 (<0.0001) | 1.25 (0.90-1.73) | 1.65 (0.19) |
| | N0/HER2- | 2.16 (1.31-3.57) | 8.60 (0.0034) | 1.61 (1.08-2.40) | 5.27 (0.022) |
| BCI | | ***Breast cancer death*** | | | |
| | N0 | 3.92 (1.98-7.76) | 17.41 (<0.0001) | 1.68 (0.94-3.01) | 3.10 (0.078) |
| | N0/HER2- | 7.18 (2.77-18.64) | 20.17 (<0.0001) | 1.78 (0.97-3.26) | 3.60 (0.057) |
| RS | N0 | 1.92 (1.46-2.52) | 16.62 (<0.0001) | 1.26 (0.86-1.84) | 1.29 (0.25) |
| | N0/HER2- | 2.54 (1.49-4.31) | 10.70 (0.0011) | 1.40 (0.88-2.23) | 1.90 (0.16) |
| IHC4 | N0 | 3.19 (2.05-4.98) | 24.02 (<0.0001) | 1.45 (0.88-2.37) | 2.04 (0.15) |
| | N0/HER2- | 4.31 (1.96-9.47) | 12.50 (0.00041) | 1.73 (0.93-3.22) | 2.83 (0.092) |
| BCI | | ***Overall survival*** | | | |
| | N0 | 1.75 (1.16-2.64) | 7.25 (0.0071) | 1.51 (1.08-2.11) | 5.86 (0.015) |
| | N0/HER2- | 1.77 (1.11-2.84) | 5.91 (0.015) | 1.54 (1.08-2.18) | 5.98 (0.014) |
| RS | N0 | 1.40 (1.12-1.75) | 7.37 (0.0066) | 1.04 (0.81-1.33) | 0.07 (0.78) |
| | N0/HER2- | 1.39 (0.97-2.01) | 2.97 (0.084) | 1.08 (0.80-1.46) | 0.25 (0.61) |
| IHC4 | N0 | 1.58 (1.15-2.17) | 7.39 (0.0066) | 1.02 (0.75-1.39) | 0.02 (0.89) |
| | N0/HER2- | 1.33 (0.81-2.20) | 1.23 (0.27) | 1.11 (0.76-1.63) | 0.30 (0.58) |

| | | | | | |
|---|---|---|---|---|---|
| ^{∗}HR was calculated as between the inter-quartile range of the continuous scores of each biomarker. Abbreviations: BCI, Breast Cancer Index; RS, OncotypeDX recurrence score; IHC4, four immunohistochemical markers (estrogen receptor, progesterone receptor, human epidermal growth factor 2, and Ki-67; HR, hazard ratio; LR-Δχ², χ² value based on the likelihood ratio statistic; CTS, clinical treatment score; NO, node negative; HER2-, epidermal growth factor receptor-negative. | | | | | |

### Node positive breast cancer

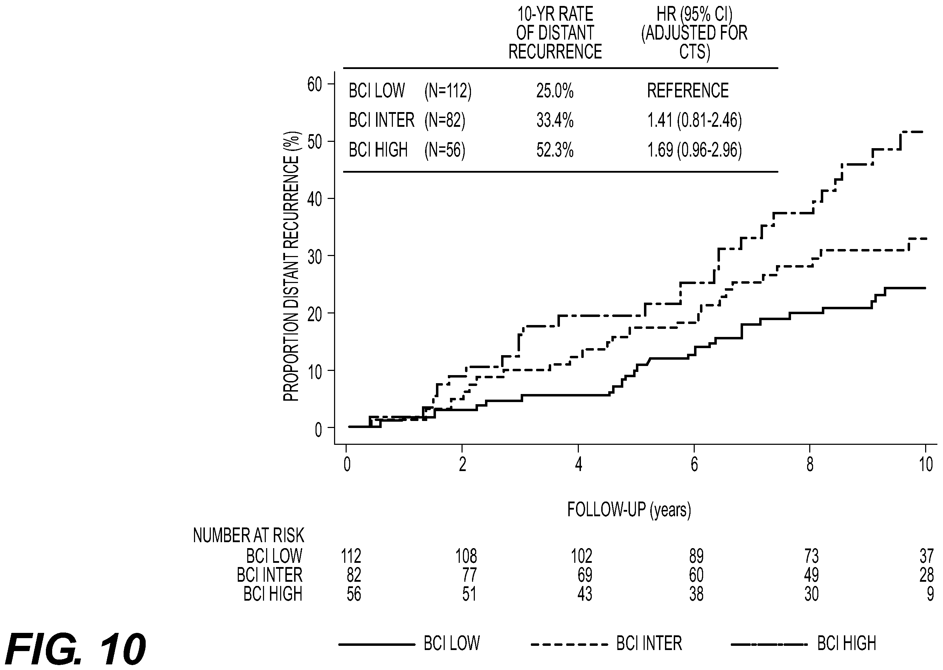

Although the primary analysis of these examples centered on NO patients, an analysis of node-positive patients showed that BCI was also prognostic for distant recurrence in these patients (log rank p=0.0045; FIG. 10). Furthermore, a comparative analysis showed that BCI, IHC4, and the 21-gene recurrence score had highly similar prognostic ability in this population of patients, albeit less robust than that noted in the NO subset (Table 5).

**Table 5. Comparative prognostic performance for 0-10 year distant recurrence of BCI, RS, IHC4 in hormone receptor-positive, node-positive patients**

| | **HR* (95% CI)** | **LR-Aχ² (P-value)** |
|---|---|---|
| **UNIVARIATE** | | |
| BCI | 1.70 (1.21-2.40) | 9.48 (0.0021) |
| RS | 1.30 (1.07-1.58) | 5.97 (0.014) |
| IHC4 | 1.40 (1.07-1.85) | 5.56 (0.018) |

| **MULTIVARIATE INCLUDING CTS** | | |
|---|---|---|
| BCI | 1.42 (1.02-1.97) | 4.49 (0.034) |
| RS | 1.25 (1.02-1.52) | 4.24 (0.039) |
| IHC4 | 1.40 (1.05-1.87) | 4.92 (0.027) |

Table 5 shows multivariate analysis in relation to cancer recurrence.

As shown by the above, BCI is prognostic of late cancer recurrences in ER+ patients following 5 years of tamoxifen treatment. *HoxB13* expression is also prognostic of late cancer recurrences in ER+ patients following 5 years of tamoxifen treatment.

### References

Cuzick et al., Lancet Oncol. 11:1135-1141 (2010).
Cuzick et al., J. Clin. Oncol. 29:4273-4278 (2011).
Dowsett et al., J. Clin. Oncol. 28:1829-1834 (2010).
Goetz et al., Clin Cancer Res. 12:2080-7 (2006).
Jansen et al., J. Clin. Oncol. 25:662-8 (2007).
Jerevall et al., Breast Cancer Res.Treat (2007).
Ma et al., Cancer Cell, 5:607-16 (2004).
Ma et al., J. Clin. Oncol., 24:4611-9 (2006).
Paik et al., N. Engl. J. Med. 351:2817-26 (2004).
Sgroi, et al., Proc SABVS; Abstract S1-9 (2012).
U.S. Patent 6,291,170
U.S. Patent 6,794,141.
U.S. Patent 7,930,105.
U.S. Patent 7,504,214.
U.S. Patent Application Publication 2005/0239079.
U.S. Patent Application Publication 2005/0239083.
U.S. Patent Application Publication 2006/0154267.
U.S. Patent Application Publication 2011/0136680.
U.S. Patent Application Publication 2013/0281502.
PCT Patent Publication WO/2012/079059.

In view of the above, it will be seen that several objectives of the invention are achieved and other advantages attained.

## Claims

1. A method of evaluating breast cancer of a subject, the method comprising measuring mRNA expression levels of *HoxB13, IL17BR, Bub1B, CENPA, NEK2, RACGAP1,* and *RRM2* in a sample of ER+ breast cancer cells from the subject; calculating a ratio of expression levels of *HoxB13*/*IL17BR* (H:I); summing the expression levels of *Bub1B, CENPA, NEK2, RACGAP1,* and *RRM2* using coefficients to obtain an MGI index; linearly combining H:I and MGI as continuous variables to form a Breast Cancer Index (BCI) value; and
classifying the sample, based on the BCI value, as indicating:
(i) a low risk or a high risk of cancer recurrence in the subject, with no intermediate risk category, wherein a higher BCI value is correlated with higher risk of cancer recurrence and a lower BCI value is correlated with lower risk of cancer recurrence; or
(ii) requirement or lack of requirement for treatment, wherein a lower BCI value is correlated with not requiring treatment with an aromatase inhibitor, targeted therapy or endocrine therapy after an initial treatment with an aromatase inhibitor, targeted therapy or endocrine therapy for five years or less.

2. The method of claim 1, wherein the coefficients are determined from principal component analysis.

3. The method of claim 1, wherein said cancer is ductal carcinoma *in situ* (DCIS) and said cancer recurrence comprises local recurrence.

4. The method of claim 1, wherein said recurrence is a recurrence more than 5 years after initial therapy.

5. The method of any one of claims 1-4, wherein:
(a) the sample is classified as indicating a high risk of distant recurrence in the subject if treated with an aromatase inhibitor, targeted, therapy, or endocrine therapy, during an initial period of 5 years or less; optionally wherein the method further comprises recommending treating the subject with chemotherapy;
(b) the sample is classified as indicating a low risk of distant recurrence in the subject if treated with an aromatase inhibitor, targeted, therapy, or endocrine therapy, during an initial period of 5 years or less; optionally wherein the method further comprises recommending treating the subject with said aromatase inhibitor, targeted, therapy, or endocrine therapy; or
(c) the sample is classified as indicating a high risk of distant recurrence in the subject if not treated with an aromatase inhibitor, targeted, therapy, or endocrine therapy, for a second 5 year period after a first 5 year period; optionally wherein the method further comprises recommending treating the subject with an aromatase inhibitor, targeted therapy, endocrine therapy, or adjuvant therapy.

6. The method of claim 1, wherein the subject is a subject that was treated for breast cancer for a period of five years or less, and further wherein the indicated requirement or lack of requirement for treatment is a treatment of five years or more.

7. The method of any preceding claim, wherein the subject has lymph-node-negative breast cancer.

8. The method of any preceding claim, wherein the subject has HER2-negative breast cancer.

9. The method of any preceding claim, wherein the sample is dissected from tissue from the subject.

10. The method of any preceding claim, wherein said sample is a formalin-fix paraffin embedded sample.

11. The method of any preceding claim, wherein the aromatase inhibitor, targeted therapy, or endocrine therapy is tamoxifen.

12. The method of claim 1, wherein the risk of recurrence is a risk of distant recurrence, a risk of local recurrence, or a risk of recurrence after 5 years.

13. The method of claim 1, wherein the low risk is a risk of cancer recurrence after receiving approximately five years of adjuvant therapy that is less than about 5%.

14. The method of claim 1, wherein low risk and high risk of cancer recurrence are distinguished relative to a selected cut-off value, optionally wherein the cut-off value is selected such that more than 50%, more than 55%, or more than 60% of ER+ subjects in a reference population are classified as low risk.

## Patentansprüche

1. Verfahren der Evaluation von Brustkrebs eines Subjekts, wobei das Verfahren Folgendes umfasst: das Messen von mRNA-Expressionsniveaus von *HoxB13, IL17BR, Bub1B, CENPA, NEK2, RACGAP1* und *RRM2* in einer Probe von ER+-Brustkrebszellen aus einem Subjekt; Berechnen eines Verhältnisses von Expressionsniveaus von *HoxB13*/*IL17BR* (H:I); Summieren der Expressionsniveaus von *Bub1B, CENPA, NEK2, RACGAP1* und *RRM2* unter Verwendung von Koeffizienten, um einen MGI-Index zu erhalten; lineares Kombinieren von H:I und MGI als kontinuierliche Variablen, um einen Brustkrebsindex (BCI) -Wert zu bilden; und
Klassifizieren der Probe, basierend auf dem BCI-Wert, als Hinweis auf:
(i) ein niedriges Risiko oder ein hohes Risiko für ein Krebsrezidiv in dem Subjekt, ohne dazwischenliegende Risikokategorie, wobei ein höherer BCI-Wert mit einem höheren Risiko für Krebsrezidiv korreliert und ein niedrigerer BCI-Wert mit einem niedrigeren Risiko für Krebsrezidiv korreliert; oder
(ii) Bedarf oder fehlender Bedarf einer Behandlung, wobei ein niedrigerer BCI-Wert damit korreliert, keine Behandlung mit einem Aromatasehemmer, gezielter Therapie oder endokriner Therapie nach einer Erstbehandlung mit einem Aromatasehemmer, gezielter Therapie oder endokriner Therapie für fünf Jahre oder weniger zu benötigen.

2. Verfahren nach Anspruch 1, wobei die Koeffizienten durch Hauptkomponentenanalyse bestimmt sind.

3. Verfahren nach Anspruch 1, wobei der Krebs ein duktales Karzinom *in situ* (DCIS) ist und das Krebsrezidiv ein Lokalrezidiv umfasst.

4. Verfahren nach Anspruch 1, wobei das Rezidiv ein Rezidiv über 5 Jahre nach Erstbehandlung ist.

5. Verfahren nach einem der Ansprüche 1-4, wobei:
(a) die Probe als ein hohes Risiko für ein entferntes Rezidiv in dem Subjekt angebend klassifiziert ist, wenn sie mit einem Aromatasehemmer, gezielter Therapie oder endokriner Therapie behandelt wird, während einer Anfangsphase von 5 Jahren oder weniger; optional wobei das Verfahren ferner das Empfehlen der Behandlung des Subjekts mit Chemotherapie umfasst;
(b) die Probe als ein niedriges Risiko für ein entferntes Rezidiv in dem Subjekt angebend klassifiziert ist, wenn sie mit einem Aromatasehemmer, gezielter Therapie oder endokriner Therapie behandelt wird, während einer Anfangsphase von 5 Jahren oder weniger; optional wobei das Verfahren ferner das Empfehlen der Behandlung des Subjekts mit dem Aromatasehemmer, gezielter Therapie oder endokriner Therapie umfasst; oder
(c) die Probe als ein hohes Risiko für ein entferntes Rezidiv in dem Subjekt angebend klassifiziert ist, wenn sie nicht mit einem Aromatasehemmer, gezielter Therapie oder endokriner Therapie behandelt wird, für einen zweiten 5-Jahres-Zeitraum nach einem ersten 5-Jahres-Zeitraum; optional wobei das Verfahren ferner das Empfehlen der Behandlung des Subjekts mit einem Aromatasehemmer, gezielter Therapie, endokriner Therapie oder adjuvanter Therapie umfasst.

6. Verfahren nach Anspruch 1, wobei das Subjekt ein Subjekt ist, das wegen Brustkrebs für einen Zeitraum von fünf Jahren oder weniger behandelt wurde, und ferner wobei der angegebene Bedarf oder fehlende Bedarf für eine Behandlung eine Behandlung von fünf Jahren oder mehr ist.

7. Verfahren nach einem vorhergehenden Anspruch, wobei das Subjekt Lymphknotennegativ-Brustkrebs hat.

8. Verfahren nach einem vorhergehenden Anspruch, wobei das Subjekt HER2-negativ-Brustkrebs hat.

9. Verfahren nach einem vorhergehenden Anspruch, wobei die Probe von Gewebe aus dem Subjekt präpariert ist.

10. Verfahren nach einem vorhergehenden Anspruch, wobei die Probe eine formalinfixierte, paraffineingebettete Probe ist.

11. Verfahren nach einem vorhergehenden Anspruch, wobei der Aromatasehemmer, die gezielte Therapie oder die endokrine Therapie Tamoxifen ist.

12. Verfahren nach Anspruch 1, wobei das Risiko für ein Rezidiv ein Risiko für ein entferntes Rezidiv, ein Risiko für ein Lokalrezidiv oder ein Risiko für ein Rezidiv nach 5 Jahren ist.

13. Verfahren nach Anspruch 1, wobei das niedrige Risiko ein Risiko für ein Krebsrezidiv nach etwa fünf Jahren adjuvanter Therapie ist, das weniger als etwa 5 % ist.

14. Verfahren nach Anspruch 1, wobei zwischen dem niedrigen Risiko und hohen Risiko für ein Krebsrezidiv relativ zu einem ausgewählten Cut-off-Wert unterschieden wird, optional wobei der Cut-off-Wert ausgewählt ist, sodass mehr als 50 %, mehr als 55 % oder mehr als 60 % von ER+-Subjekten in einer Referenzpopulation als mit geringem Risiko klassifiziert sind.

## Revendications

1. Procédé d'évaluation du cancer du sein d'un sujet, le procédé comprenant la mesure des niveaux d'expression d'ARNm de *HoxB13, IL17BR, Bub1B, CENPA, NEK2, RACGAP1* et *RRM2* dans un échantillon de cellules de cancer du sein ER+ du sujet ; le calcul d'un rapport des niveaux d'expression de *HoxB13* / *IL17BR* (H : I) ; l'addition des niveaux d'expression de Bub1B, *CENPA, NEK2, RACGAP1* et *RRM2* en utilisant des coefficients pour obtenir un indice MGI ; la combinaison linéaire de H : I et MGI en tant que variables continues pour former une valeur d'indice de cancer du sein (BCI) ; et
le classement de l'échantillon, sur la base de la valeur BCI, comme indiquant :
(i) un risque faible ou un risque élevé de récidive du cancer chez le sujet, sans catégorie de risque intermédiaire, dans lequel une valeur BCI supérieure est corrélée à un risque plus élevé de récidive du cancer et une valeur BCI inférieure est corrélée à un risque plus faible de récidive du cancer ; ou
(ii) l'exigence ou l'absence d'exigence de traitement, dans lequel une valeur BCI inférieure est corrélée avec l'absence d'exigence de traitement avec un inhibiteur d'aromatase, une thérapie ciblée ou une thérapie endocrinienne après un traitement initial avec un inhibiteur d'aromatase, une thérapie ciblée ou une thérapie endocrinienne pendant cinq ans ou moins.

2. Procédé selon la revendication 1, dans lequel les coefficients sont déterminés à partir d'une analyse en composantes principales.

3. Procédé selon la revendication 1, dans lequel ledit cancer est un carcinome canalaire *in situ* (DCIS) et ladite récidive de cancer comprend une récidive locale.

4. Procédé selon la revendication 1, dans lequel ladite récidive est une récidive plus de 5 ans après la thérapie initiale.

5. Procédé selon l'une quelconque des revendications 1 à 4, dans lequel :
(a) l'échantillon est classé comme indiquant un risque élevé de récidive à distance chez le sujet s'il est traité avec un inhibiteur d'aromatase, une thérapie ciblée, ou une thérapie endocrinienne au cours d'une période initiale de 5 ans ou moins ; éventuellement dans lequel le procédé comprend en outre la recommandation de traiter le sujet par chimiothérapie ;
(b) l'échantillon est classé comme indiquant un faible risque de récidive à distance chez le sujet s'il est traité avec un inhibiteur d'aromatase, une thérapie ciblée, ou une thérapie endocrinienne pendant une période initiale de 5 ans ou moins ; éventuellement, dans lequel le procédé comprend en outre la recommandation de traiter le sujet avec ledit inhibiteur d'aromatase, ladite thérapie ciblée, ou ladite thérapie endocrinienne ; ou
(c) l'échantillon est classé comme indiquant un risque élevé de récidive à distance chez le sujet s'il n'est pas traité avec un inhibiteur d'aromatase, une thérapie ciblée, ou une thérapie endocrinienne pendant une seconde période de 5 ans après une première période de 5 ans ; éventuellement dans lequel le procédé comprend en outre la recommandation de traiter le sujet avec un inhibiteur d'aromatase, une thérapie ciblée, une thérapie endocrinienne ou une thérapie adjuvante.

6. Procédé selon la revendication 1, dans lequel le sujet est un sujet qui a été traité pour un cancer du sein pendant une période de cinq ans ou moins, et en outre dans lequel l'exigence indiquée ou l'absence d'exigence de traitement est un traitement de cinq ans ou plus.

7. Procédé selon l'une quelconque des revendications précédentes, dans lequel le sujet a un cancer du sein à ganglions lymphatiques négatifs.

8. Procédé selon l'une quelconque des revendications précédentes, dans lequel le sujet a un cancer du sein HER2 négatif.

9. Procédé selon l'une quelconque des revendications précédentes, dans lequel l'échantillon est disséqué à partir du tissu du sujet.

10. Procédé selon l'une quelconque des revendications précédentes, dans lequel ledit échantillon est un échantillon incorporé à la paraffine fixant du formol.

11. Procédé selon l'une quelconque des revendications précédentes, dans lequel l'inhibiteur d'aromatase, la thérapie ciblée ou la thérapie endocrinienne est le tamoxifène.

12. Procédé selon la revendication 1, dans lequel le risque de récidive est un risque de récidive à distance, un risque de récidive locale, ou un risque de récidive après 5 ans.

13. Procédé selon la revendication 1, dans lequel le faible risque est un risque de récidive du cancer après avoir reçu environ cinq ans de traitement adjuvant qui est inférieur à environ 5%.

14. Procédé selon la revendication 1, dans lequel un faible risque et un risque élevé de récidive du cancer sont distingués par rapport à une valeur seuil sélectionnée, éventuellement dans laquelle la valeur seuil est choisie de telle sorte que plus de 50%, plus de 55%, ou plus de 60% des sujets ER+ dans une population de référence sont classés comme faible risque.
